# EUROPEAN PATENT SPECIFICATION

(11) **EP 0 578 653 B1**
(45) Date of publication and mention of the grant of the patent: **18.07.2001**
(21) Application number: 92906427.7
(22) Date of filing: 12.03.1992
(51) Int. Cl.: C12N 15/00, C12N 15/85, C12N 15/18, A01K 67/027, C12Q 1/68, C07K 14/00

(54) **GENE CONSTRUCT FOR PRODUCTION OF TRANSGENIC FISH**
GENHERSTELLUNG ZUR PRODUKTION VON TRANSGENFISCH
CONSTRUCTION GENIQUE DESTINEE A LA PRODUCTION DE POISSONS TRANSGENIQUES

(30) Priority: 15.03.1991 US 669765
(43) Date of publication of application: 19.01.1994
(73) Proprietor: HSC RESEARCH DEVELOPMENT LIMITED PARTNERSHIP, Toronto Ontario, M5G 1X8 (CA); SEABRIGHT CORPORATION LIMITED, St. John's, Newfoundland, A1C 5S7 (CA)
(72) Inventor: HEW, Choy, L., Thornhill, Ontario L4J 2A3 (CA); FLETCHER, Garth, L., St. John's, Newfoundland ALC 5H4 (CA)
(74) Representative: Thomson, Paul Anthony
(86) International application number: PCT/CA92/00109
(87) International publication number: WO 92/16618

(56) References cited:
- BIOTECHNOLOGY vol. 10, no. 2, February 1992, NEW YORK US pages 176 - 181; DU, S. J. ET AL.: 'Growth enhancement in transgenic Atlantic salmon by the use of an "all fish" chimeric growth hormone gene construct' cited in the application
- JOURNAL OF BIOLOGICAL CHEMISTRY. vol. 263, no. 24, 25 August 1988, BALTIMORE US pages 12049 - 12055; HEW, C. L. ET AL.: 'Multiple genes provide the basis for antifreeze protein diversity and dosage in the Ocean pout, Macrozoarces americanus' cited in the application
- MOL. MAR. BIOL. BIOTECHNOLOGY vol. 1, no. 1, September 1991, pages 58 - 63; SHEARS, M.A. ET AL.: 'Transfer, expression, and stable inheritance of antifreeze protein genes in Atlantic salmon (Salmo salar)' cited in the application
- MOL: MAR. BIOL. BIOTECHNOL. vol. 1, no. 1, September 1991, pages 64 - 72; GONG, Z. ET AL.: 'Functional analysis and temporal expression of promoter regions from fish antifreeze protein genes in transgenic Japanese medaka embryos' cited in the application

## Description

### FIELD OF THE INVENTION

This invention relates to transgenic fish and an "all fish" promoter sequence useful in developing transgenic fish.

### BACKGROUND OF THE INVENTION

Throughout the specification, several articles are referred to in order to provide complementary information regarding the invention. The complete citations for those references are provided below:
Agellon, L. B. and Chen. T. T., (1986) Rainbow trout growth hormone : Molecular cloning of cDNA and expression in E. coli. *DNA,* 5:463-477.
Agellon, L.B., Emery, C.J., Jones, J.M., Davies, S.L., Dingle, A.D. and Chen, T.T. (1988) Promotion of rapid growth of rainbow trout (Salmo gairdneri) by a recombinant fish growth hormone. *Can. J. Fish. Auqatic Sci*. 45:146-151.
Cantilo, E. and Regalado, T. G. (1942) Investigaciones realizadas con el extracto anterohysofisario en el desarrollo del Salvelinus Fontinalis. *Rev. Med. Vet.* (Buenos Aires) 24:323-338.
Chen, T. T., Lin, C. M., Zhu, Z., Gonzalez-Villasenor, L. I., Dunham, R. A., and Powers, D. A. (1990) Gene transfer, expression and inheritance of rainbow trout and human growth hormone genes in carp and loach. pp 127-139 *In Transgenic Models in Medicine and Agriculture,* Wiley-Liss, Inc, N.Y..
Chen, S. and Evans, G. A. (1990) A simple screening method for transgenic mice using the polymerase chain reaction. *Biotechniques,* 8:32-33.
Chong, S. S. C., and Vielkind J. R. (1989) Expression and fate of CAT reporter gene microinjected into fertilized medaka (Oryazias latipes) eggs in the form of plasmid DNA, recombinant phage particles and its DNA. *Theor. Appl*. *Genet.* 78 : 369-380.
Connelly, S. and Manley, J. L. (1988) A functional mRNA polyadenylation signal is required for transcription termination by RNA polymerase II. *Gen. Dev.* 2:440-452.
Davies, L. G., Dibuer, M. D. and Battey, J. F. (1986) Basic Methods in Molecular Biology. *Elsevier Science Publishing Co.*
Davies, P. L., Fletcher, G. L. and Hew, C. L. (1989) Fish antifreeze protein genes and their use in transgenic studies. in: *Oxford Surveys on Eukaryotic Genes,* 6: 85-110. Edited by Norman Maclean, Published by Oxford University Press.
Davies, P. L. and Hew, C. L. (1990) Biochemistry of fish antifreeze proteins. *The FASEB Journal,* 4:2460-2468.
De Simone, V. and Cortese, R. (1988) The transcriptional regulation of liver-specific gene expression. p. 51-90. *In: Oxford surveys on eukarytotic genes.* N. Maclean (Ed.) Oxford University Press, NY.
Du, S. J. and Hew, C. L. (unpublished) The isolation and characterization of GH genes from Atlantic salmon and Chinook salmon.
Du, S. J., Gong, Z., Fletcher, G. L., Shears, M. A., King, M. J., Idler, D. R. and Hew, C. L. (1992) Growth enhancement in transgenic Atlantic salmon by the use of an "all fish" chimeric growth hormone gene construct. *Bio/Technology*, 10:176-181.
Fletcher, G. L., Hew, C. L., Li, X., Haya, K. and Kao, M. H. 1985. Year-round presence of high levels of plasma antifreeze peptides in a temperate fish, ocean pout *(Macrozoarces americanus).* Can. J. Zool. 63:488-493.
Fletcher, G. L. and Davies, P. L. (1991) Transgenic fish for aquaculture. p. 331-370. In: Genetic Engineering, Principles and methods. *J. K*. *Setlow* (Ed.). Plenum Press, NY.
Fletcher, G. L., Shears, M. A., King, M. J., Davies, P. L. and Hew, C. L. (1988) Evidence for antifreeze protein gene transfer in Atlantic salmon (Salmo Salar). *Can*. *J. Fisheries and Aquatic Sciences,* 45:352-357.
Fletcher, G. L., Idler, D. R., Vaisius, A. and Hew, C. L. (1989) Hormonal regulation of antifreeze protein expression in winter flounder. *Fish Physiology and Biochemistry,* 7:387-393.
Friedenreich, H. and Schartl, M. (1990) Transient expression directed by homologous and heterologous promoter and enhancer sequences in fish cells. *Nucleic Acids Research,* 18:3299-3305.
Gill, J. A., Sumpter, J. P., Donaldson, E. M., Souza, L., Berg, T., Wypych, J. and Langley, K. (1985) Recombinant chicken and bovine growth hormones accelerate growth in aquacultured juvenile Pacific salmon, (Oncorhynchus kisutch). *Bio/Technology*, 3:643-646.
Gong, Z., Fletcher, G. L. and Hew, C. L. (1992) Tissue distribution of antifreeze mRNA. *Can. J. Zool.* In press.
Gong, Z. Y., Hew, C. L. (unpublished) Functional analysis of fish antifreeze gene promoters: presence of silencers.
Gong, Z. Y., Vielkind, J., Hew, C. L., (1991) Functional analysis and temporal expression of fish antifreeze gene promoters in Japanese medaka embryos. *Mol*. *Marine Biol*. *Biotech.* 1:64-72
Guyomard, R., Chourrout, D., Leroux, C., Houdebine, L. M. and Pourrain, F. (1989) Integration and germ line transmission of foreign genes microinjected into fertilized trout eggs. *Biochimie*, 71:857-863.
Hammer, R. E., Brinster, R. L. and Palmiter, R. D. (1985) Use of gene transfer to increase animal growth. *Cold Spring Harbor Symp*. *Quant*. *Biol.* 50:379-388.
Hanley, T., and Merlie, J. P. (1991) Transgene detection in unpurified mouse tail DNA by polymerase chain reaction. *BioTechniques.* 10:56-56.
Hew, C. L., Slaughter, D., Joshi, S. B., and Fletcher, G. L. (1984) Antifreeze polypeptides from the Newfoundland ocean pout, Macrozoarces americanus : Presence of multiple and compositionally diverse components. *J. Comparative Physiology B,* 155:81-88.
Hew, C. L., Wang, N. C., Joshi, S., Fletcher, G.L., Scott, G. K., Hayes, P. H., Buettner, B. and Davies, P. L. (1988) Multiple genes provide the basis for antifreeze protein diversity and dosage in the Ocean Pout (Macrozoares americanus). *J. Biol*. *Chem*., 263:12049-12055
Hew, C. L., Trinh, K. Y., Du, S. J., and Song, S. D. (1989) Molecular cloning and expression of salmon pituitary hormones. *Fish Physiology and Biochemistry,* 7:375-380.
Hew. C. L. (1989) Transgenic fish: present status and future directions. *Fish Physiol. Bioch*. 7:409-413.
Hoar, W. S. (1988) The physiology of smolting salmonids. in *Fish Physiology,* vol. 11:275-343. edited by Hoar, W. S. and Randall, D. J. Academic Press.
Jankowski, J. M. and Dixon, G. H. (1987) The GC box as a silencer. *Bioscience reports* 7:955963.
Kawauchi, H. Moriyama, S., Yasuda, A., Yamaguchi, K. Shirahata, K., Kubota, J. and Hirano, T. (1986) Isolation and characterization of Chum salmon growth hormone. *Archives of Biochemistry and Biophysics,* 244:542-552.
Li, X., Trinh, K., Hew, C. L., Buettner, B., Baenziger, J. and Davies, P. L. (1985) Structure of an antifreeze polypeptide and its precursor from the ocean pout, Macrozoarces americanus. *J. Biol*. *Chem.* 260:12904-12909.
Liu, Z., Moav, B., Faras, A. J., Guise, K. S., Kapuscinski, A. R. and Hackett, P. B. 1990a. Functional analysis of elements affecting expression of the *β*-actin gene of carp. *Mol*. *Cell*. *Biol.* 10:3432-3440.
Liu, Z., Moav, B., Faras, A. J., Guise, K. S., Kapuscinski, A. R. and Hackett, P. B. (1990b) Development of expression vectors for transgenic fish. *Bio/Technology* 8:1268-1272.
Luckow, B., and Schutz, G. (1987) CAT construction with multiple unique restriction sites for the functional analysis of eucaryotic promoters and regulatory elements. *Nucleic Acids Res.,* 15:5490.
McHale, R. H., Stapleton, P. M., and Bergquist, P. L. (1991) Rapid preparation of blood and tissue samples for polymerase chain reaction. *BioTechniques.* 10:20-22.
Maclean, N., Penman, D. and Zhu, Z. (1987) Introduction of novel genes into fish. *Bio/Technology* 5:257-261.
Maclean, N. and Penman, D. (1990) The application of gene manipulation to aquaculture. *Aquaculture* 85:1-20.
Palmiter, R. D., Brinster, R. L., Hammer, R. E., Trumbauer, M. E., Rosenfeld, M. G., Birnberg, N. C. and Evans, R. M. (1982) Dramatic growth of mice that develop from eggs microinjected with metallothionein-growth hormone fusion genes. *Nature*, 300:611-615.
Proudfoot, N. J. (1989) How RNA polymerase II terminates transcription in higher eukaryotes. *TIBS*. 14:105-1 10.
Rokkones, E., Alestrom, P., Skjervold, H. and Gautvik, K. M. (1989) Microinjection and expression of a mouse metallothionein human growth hormone fusion gene in fertilized salmonid eggs. *J. Comp. Physiol. B.,* 158:751-758.
Schorpp, M., Kugler, W., Wagner, U. and Ryffel, G. U. 1988. Hepatocyte-specific promoter element HPI of the xenopus albumin gene interacts with transcriptional factors of mammalian hepatocytes. *J. Mol*. *Biol.* 202:307-320.
Sekine, S., Mizukami, T., Nishi, T., Kuwana, Y., Saito, A., Sato, M., Itoh, S., and Kawauchi, H. (1985) Cloning and expression of cDNA for salmon growth hormone in E. coli. *Proc*. *Natl*. *Acad*. *Sci. U*. *S*. *A.,* 82:4306-4310.
Shears, M. A., Fletcher, G. L. Hew, C. L. Gauthier, S. and Davies, P. L. (1991) Transfer, expression, and stable inheritance of antifreeze protein genes in Atlantic salmon *(Salmo salar)*. *Mol*. *Marine Biol*. *Biotech.* 1:58-63.
Tuckmann, H. (1936) Action de l'hypophyse sur la morphogenese et la differentiation sexuselle de Girardinus Guppii. *C.R. Soc*. *Biol.* 122:162-164
Vielkind, J., Haas-Andela, H., Vielkind, U. and Anders, F. (1982) The induction of a specific pigment cell type by total genomic DNA injected into the neural crest region of fish embryos of genus (Xiphophorus). *Mol. Gen. Genet.* 185 : 379-389.
Vize, P. D., Michalska, A. E., Ashman, R., LLoyd, B., Stone, B. A., Quinn, P., Wells, J. R. E. and Seamark, R. F. (1988) Introduction of a porcine growth hormone fusion gene into transgenic pigs promotes growth. *J. Cell Science,* 90 : 295-300.
Yanisch-Perron, C., Vieira, J. and Messing, J. (1985) Improved M13 phage cloning vectors and host strains: nucleotide sequences of the M13mp18 and pUC19 vectors. *Gene* 33:103-119.
Zafarullah, M., Bonham, K. and Gedamu, L. (1988) Structure of the rainbow trout metallothionein B gene and characterization of its metal-responsive region. *Mol*. *Cell Biol.,* 8:4469-4476.
Zhang, P., Hayat, M., Joyce, C., Gonzalezvillasenor, L. I., Lin, C. M., Dunham, R. and Chen, T. T. and Powers, D. A. (1990) Gene transfer, expression and inheritance of pRSV-rainbow trout GHcDNA in the common carp, Cyprinus carpio (Linnaeus). *Molecular Reproduction and Development,* 25:3-13.
Zhou, J. A., McIndoe, A., Davies, H., Sun, X. Y. and Crawford, L. (1991) The induction of cytotoxic T-lymphocyte precursor cells by recombinant vaccinia virus expressing human papillomavirus type 16 Ll. *Virology* 181:203-210.
Zhu, Z., Liu., G., He, L. and Chen, S. (1985) Novel gene transfer into fertilized eggs of goldfish (Carassius auratus L. 1758). *Z*. *Angew*. *Ichthyol.,* 1:31-34.
Zhu, Z., Xu, K., Li, G., Xie, Y. and He, L. (1986) Biological effects of human growth hormone gene microinjected into the fertilized eggs of loach (Misgurnus anguillicaudatus). *Kexue Tongbao,* 31:988-990.

A variety of attempts and successes have been made in developing transgenic fish and other animals. Growth hormone has been of particular interest in past investigations. Growth hormone is a single chain polypeptide hormone that plays a principal role in the regulation of somatic growth and development in animals. Many approaches have been made to increase fish growth by growth hormone. These include the feeding of pituitary extracts (Tuckmann 1936, Cantilo and Regalado 1940), injection or implantation of purified recombinant-derived growth hormone (Gill et al. 1985, Sekine et al. 1985, Kawauchi et al. 1986, Agellon et al. 1988). All these results clearly showed that growth hormone alone is effective in stimulating fish growth. However, all these studies have limited application in aquaculture, and one major drawback is that the phenotype can not be inherited.

Gene transfer technique has become a new and powerful approach to manipulate the genetic and phenotypic characteristic of both animals and plants. Various reports have been made in the production of transgenic fish. The first transgenic study on fish was reported by Vielkind et al. (1982). These investigators injected swordtail tumour genes into the Platyfish, and found that the injected swordtail Tu genes could induce T-melanophore induction in Tu-free Platyfish. In 1985 and 1986, Zhu et al. reported the production of transgenic fish by growth hormone gene transfer. Using a mouse metallothionein promoter ligated to a human GH structural gene, they successfully produced transgenic loach, goldfish and silver carp. On the average, the transgenic fish was 1 to 3 times larger than control. Since then, several reports using similar gene constructs have been published (Rokkones et al. 1989, Guyomard et al. 1989, Chen et al. 1990). Further work has also been reported in Maclean et al 1987; Hew 1989; Maclean and Penman 1990 and Fletcher and Davies 1991. The earlier GH gene transfer studies on fish were made by using mammalian metallothionein promoters or viral promoters and human or rat GH genes. (Zhu et al. 1986, Rokkones et al. 1989, Guyomard et al. 1989 and Chen et al. 1990). There are two problems associated with using those heterologous gene constructs. First, the transgenic fish produced using mammalian GH genes may not be suitable or acceptable for human consumption. Secondly, it has been reported by Friedenreich and Schartl (1990) that the mammalian GH gene could not be spliced sufficiently in fish cell line in vitro, and they could not detect the expression of the GH gene in fish cells. This may explain why Rokkones et al. (1989) and Guyomard et al (1989) could not observe faster growth in their transgenic fish by using mammalian metallothioneinmammalian GH fusions or viral promoter-mammalian GH genes. Zhang et al. (1990) and Chen et al. (1990) used the rainbow trout GH gene for gene transfer in Carp and Loach, using the retrovirus promoter. However, the transgenic fish in those investigations were only 20% larger than the controls, and the trout GH gene used lacked the signal sequence needed for the proper secretion and action of GH.

Most, if not all of these studies were carried out by using either mammalian GH or mammalian gene and viral promoters. To be acceptable in aquaculture, the promoter(s) and gene(s) used in transgenic fish should be derived preferably from fish protein genes to avoid the possibility of any potential health hazards. Furthermore the production of a strain of faster growing fish in an economically important species such as salmonids with an "all fish" gene construct will be beneficial to fish farming.

Recently, an "all fish" expression vector using the carp beta-actin promoter has been published (Liu et al., 1990b). One problem of great concern with this vector is that the vector uses the polyadenylation signal from the chinook salmon GH cDNA (Hew et al., 1989) as the transcription termination signal. However, transcription termination requires both a functional polyadenylation signal and a GT-rich downstream element (Connelly and Manely, 1988; Proudfoot, 1989). Therefore, the polyadenylation signal of the GH cDNA is most likely insufficient to function as a transcription termination signal. Secondly, the beta-actin promoter is expressed in most if not all tissues or laks tissue-specificity.

### SUMMARY OF THE INVENTION

According to the present invention there is provided a transgenic fish characterised by having incorporated in its genome a chimeric gene construct wherein said chimeric gene construct compriese:
a type III AFP promoter; and
a fish growth hormone gene sequence.

In a further aspect of the present invention there is provided a promoter sequence/transcriptional terminal sequence for use in constructing a chimeric gene construct for incorporation in the genome of a transgenic fish in accordance with the present invention, said combined sequence including a 5' untranslated sequence between said promoter and terminal sequences, said combined sequences having the sequence of Table V with unique restriction sites of Bg1II at base pair position 2116 and of HpaI at base pair position 2188.

Further the present invention provides a chimeric gene construct for incorporation in fish genome to produce a transgenic fish in accordance with the present invention, wherein said chimeric gene construct comprises a promoter which has the DNA sequence of Table V from 5' at base pair position 1 to base position 2115.

Additionally, the present invention provides a chimeric gene construct for incorporation in fish genome to produce a transgenic fish according to the present invention, wherein said chimeric gene construct comprises a transcriptional terminal sequence wherein said sequence has the DNA sequence of Table V from base pair position 2188 to the 3' at base pair position 3350.

Furthermore, the present invention also provides a fish host transformed with a chimeric gene in accordance with the present invention.

The invention provides specific embodiments, such as the analysis of transient expression of the OP-AFP gene promoter activities in a salmonid cell line and the Japanese medaka embryos, the construction of AFP-GH fusion gene, and its gene transfer by microinjection, screening of transgenic salmon by polymerase chain reaction (PCR), and size and growth rate measurements.

### BRIEF DESCRIPTION OF THE DRAWINGS

Figure 1. (A). Construction of pOP-CAT for transient CAT assay in salmonid cell lines and Japanese medaka embryos. The plasmid is named as pOP5B. (B). Construction of all fish chimeric gene (pOP-GHe) for gene transfer;
Figure 2. Strategy of PCR analysis. Three sets of primers were used to detect the presence of transgene. The distance between primers are 813 bp for primer A and B, 335 bp for primers A and D, 119 bp for primers C and D (sequencing sutdies gave more refined data for the distance between primers; i.e., 855 bp for primer A and B; 333 bp for primers A and D; 199 bp for primers C and D);
Figure 3. Analysis of the OP-AFP promoter activity in rainbow trout hepatoma cells by CAT assay. The ocean pout-CAT plasmid is pOP5B;
Figure 4. Time course analysis of CAT expression in embryos injected with OP-CAT (pOP%B). A pool of five embryos were used for CAT assay of 1 day, 2 day, 4 day, 6 day, 8 day and 11 day embryos. Individual larva was used for the CAT assay of hatched medaka (one day after hatching). Embryos injected with pBLCAT3 was used as negative control;
Figure 5. Screening of transgenic salmon by PCR using primers A/B. (A). Analysis of PCR amplified products by agarose gel electrophoresis. (B). Southern blot analysis of the PCR product by using GH specific probe E;
Figure 6. (A). Confirming the transgenic salmon by PCR using primers C/D. (B). Study of the integrity of the transgene by PCR using primers A/B;

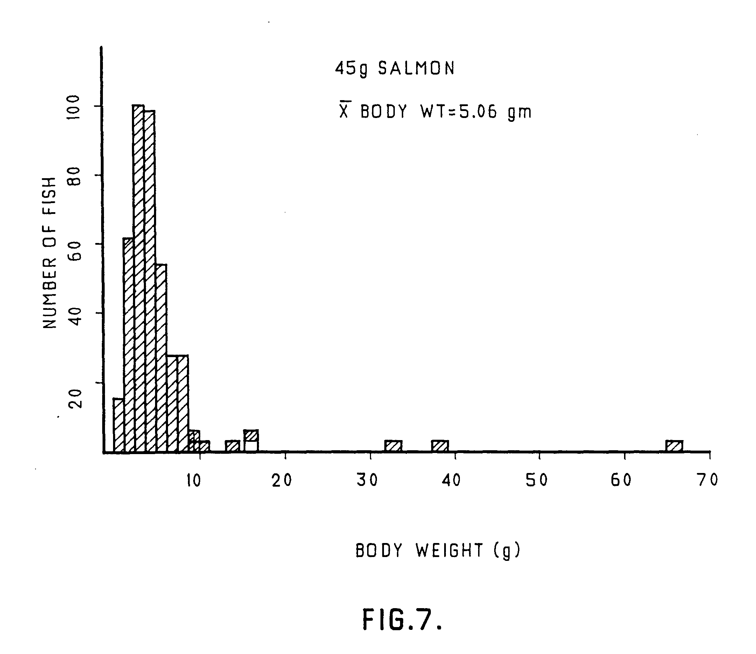

Figure 7. The size distribution of transgenic salmon and nontransgenic salmon;
Figure 8. Transgenic salmon vs nontransgenic salmon;
Figure 9. Analysis of the opAFP gene promoter activity by CAT assay in two salmoid cell lines, which include the rainbow trout hepatoma cell line RTH-149 and the chinook salmon embryonic cell line CHSE-214. The acetylated and non-acetylated chloramphenicols, as indicated by AC and C on the right, were separated by thin layer chromatography, followed by autoradiogaphy;
Figure 10. CAT expression in different stages of embryos injected with opAFP-CAT. Individual embryo was used for each CAT assay. The acetylated and non-acetylated chloramphenicols are indicated by AC and C on the right. The time points analyzed were day 1, day 2, day 6, day, 9 and day 11;
Figure 11 is a diagram of opAFP-V. ▭ ocean pout AFP gene promoter and gene 3'-flanking sequences; 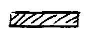 ocean pout AFP gene 5'-untranslated sequence; 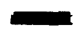 , ocean pout AFP gene 3'-untranslated sequence; _____________, pUC plasmid DNA sequence and 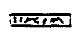 , Ampicillin resistance gene. The TATA box, the poly(A) signal (AATAAA) and the predicted transcription termination signal (TTTTTCT) are indicated. Restriction sites are: A, AatII; Ba, BamHI; Bg, BglII; E, EcoRI; H, HindIII; Hp, Hpal; K, Kpnl; Sac, Sacl; Sal, Sall and Sm, Smal;
Figure 12. Transgenic salmon (#34, 33 g) and nontransgenic siblings (average weight 5-7 g); and
Figure 13. Size-frequency distribution of salmon. (A) Fish (200) were weighed in October 1990 and 50 were tagged and blood sampled for DNA analysis (solid bars).
   The presence of individual transgenic fish is identified by T. (T) indicates a transgenic fish that was found to be posisible by Scale DNA analysis only. (B) All fish (484) in the aquarium were weighed in January 1991 and blood sampled for DNA analysis. T indicates the presence of a transgenic salmon. (T) indicates a transgenic fish that was found to be positive by scale DNA analysis only.

### DETAILED DESCRIPTION OF THE PREFERRED EMBODIMENTS

The present invention provides the successful production of transgenic fish with dramatic expression of the desired genetic trait. According to one embodiment of the invention, a chimeric gene, pOP-GHe was constructed by using antifreeze promoter linked to the chinook salmon GH cDNA clone. This gene construct pOP-GHe was microinjected into fertilized, nonactivated Atlantic salmon eggs via the micropyles. Transgenic Atlantic salmon carrying the transgene were generated with an incorporation frequency of at least 2%. The presence of transgene was detected by polymerase chain reaction using specific oligonucleotide primers. These transgenic fish showed dramatic increase in their weight and growth rate. At eight months old, the average increase of the transgenic fish was 4-fold and the largest transgenic fish was eight times bigger than the non-transgenic controls. These studies demonstrated that the AFP promoter was effective in producing large transgenic salmon and would be applicable to many different species of fish. Use of fish growth hormone with promoter of this invention will result in transgenic fish up to eight times larger than controls. This is the largest increase that has been reported to date for a transgenic fish. Comparing with the 2-fold increase in transgenic mouse (Palmiter et al. 1982) and transgenic pig (Vize et al. 1988), these studies indicate that growth of transgenic salmon is more pronounced.

The data later presented in this specification demonstrate further the successful production of transgenic salmon by using a DNA construct derived from fish genes. In one aspect of our development of the invention, ocean pout antifreeze gene promoter and transcription termination signal were ligated with chinook salmon GH gene, both of which are derived from fish genes, therefore avoiding the problems associated with the use of mammalian or viral genes for gene transfer in commercially important fish.

The following abbreviations are used:
- opAFP: Ocean pout antifreeze proteins.
- OpAFP-CAT: A chimeric plasmid consisting of the ocean pout antifreeze protein promoter linked to the bacterial chloraphenicol acetyl transferase. (Originally this product was referred to as "pOP-CAT. During further development, we chose to identify it as "opAFP-CAT").
- opAFP-GHc: An "all fish" chimeric gene consisting of the ocean pout antifreeze protein promoter and 3' non-translated sequences linked to the fish growth hormone cDNA gene. (Originally this product was referred to as "pOP-GHe. During further development, we chose to identify it as "opAFP-GHc").
- opAFP-V: An "all fish" gene cassette based on the use of the ocean pout antifreeze promoters and 3'non-translated sequences.

The 2kb BamHI - BglII fragment of OP-AFP characteristic of the promoter was shown by sequencing studies to have a length of 2.1 kb. The 3' sequence HpaI - HindIII fragment was shown to have a length of 1.1 kb.

The ocean pout AFP is a member of the type III AFP (Davies and Hew 1990). It has approximately 150 gene copies (Hew et al. 1988, Davies et al. 1989). The protein and genomic structure of this AFP have been well characterized by us (Hew et al. 1984, 1988, Li et al. 1985, 1989). More recently, its promoter sequences have been investigated in both the salmonid cell lines and the Japanese medaka embryos (Gong and Hew unpublished, Gong et al. 1991. See Table II). The ocean pout AFP gene is expressed predominantly in liver cells (Gong et al. 1991), all of these manuscripts being incorporated herein by reference.

Unlike the type I, alanine-rich AFP from the winter flounder which is synthesized only in the winter, the ocean pout AFP is present all year around, albeit at a higher concentration during the winter months (Fletcher et al. 1989). The following data demonstrate that the OP-AFP promoter is a very effective promoter for inducing CAT and GH gene or other desired compatible gene expression in fish, such as Atlantic salmon. Although there is no antifreeze (AFP) gene in salmonids or medaka genomes, it is likely in view of successful implementation of the invention that the transcriptional factors controlling the OP-AFP gene expression do exist in salmon and in all other fish. This is consistent with our earlier investigation in producing the transgenic Atlantic salmon using the type I antifreeze protein gene from the winter flounder (Fletcher et al. 1988). In that investigation, a DNA coding for the genomic sequence of the AFP gene and its 5' and 3' flanking sequences were used. Transgenic flounder animals and Fl generations producing circulating AFP in the serum were achieved (Shears et al. 1991). Our studies of the ocean pout promoter in the salmonid cell lines, the Japanese medaka embryos and the positive results from the transgenic Atlantic salmon indicate that the promoter is useful in a variety of fish species. The present AFP gene construct can be further developed into a gene cassette where many other fish genes of interest can be inserted.

The ocean pout AFP gene promoter is attractive in several respects. First of all, it is expressed predominantly in the liver, a tissue that has large synthetic and secretory capabilities. The expression of a transgene in liver is one of the most common approaches in gene transfer studies. Secondly, the AFP gene is present only in a small number of fish species. Its expression will not be affected by the host genome, because there are no homologous endogenous genes. Moreover, the absence of the AFP gene in most fish genomes makes the detection of this AFP derived gene construct simple without any background contributed from the host DNA. This was clearly illustrated in our studies of transgenic Atlantic salmon (Du et al., 1992). Finally, it has been shown that there are several positive and negative cis-acting elements in the opAFP gene promoter (Gong et al. 1991). This provides an opportunity to modify this vector in order to meet the different requirements of transgene expression.

We have also determined that adequate expression of the selected gene can be achieved by placing the 1.2 kb of opAFP gene 3' sequence in our vector opAFP-V. DNA sequencing analysis shows that it contains a typical polyadenylation signal AATAAA. Moreover, a 7 bp sequence motif (TTTTTCT) was found 26 bp downstream of the poly(A) signal. This 7 bp motif is a suitable transcription termination signal (TTTTTNT) as in virus (Zhou et al., 1991). The 1.2 kb sequence can therefore function as a general transcription termination signal in gene expression.

The development of the opAFP gene cassette; i.e. opAFP-V, has greatly simplified the insertion of other genes for gene transfer. Transgenic salmon and other species of trangenic fish can be produced with other pituitary hormones and many other proteins and polypeptides using this expression vector.

### EXPERIMENTAL PROCEDURES - FIRST SERIES OF EMBODIMENTS OF THE INVENTION

### A-1 Atlantic salmon eggs collection

Mature Atlantic salmon (Salmo salar) were captured 2-3 weeks prior to spawning from the exploits and Colinet river systems, Newfoundland, and transported live to the ocean Sciences Centre, Memorial University of Newfoundland. The fish were maintained at seasonally ambient photoperiod in 2 x 2 x 0.5 m aquaria supplied with freshwater and air.

Eggs and sperms were stripped from salmon which had been anaesthetized in a dilute solution of t-amyl alcohol. Eggs were kept in 4°C, and were fertilized up to 2h prior to microinjection and rinsed with several changes of ice-cold salmon Ringer solution. Activation occurred when the eggs were placed in fresh water after microinjection (Fletcher et al. 1988).

### A-2 Medaka egg collection

Japanese medaka (Oryzias latipes) were maintained at Dr. J. Vielkind's laboratory, cancer research centre, Vancouver. The reproductive activities of the adults were induced with artificial photoperiod of 10 hours darkness to 14 hours light. Fertilized eggs attached to females were collected 1-2 h after the onset of light and maintained in Ringer solution (0.75% NaCl, 0.02% KCl, 0.02% CaC₂l:, pH 7.3) at 12°C prior to injection. Injected medaka embryos were reared in medium containing 0.1% NaCl, 0.003% KCl, 0.004% CaCl₂:.2H₂0, 0.016% MgSO₄.7H₂O, 0.0001% methylene blue and transferred to aquarium water immediately after hatching (Chong and Vielkind 1989).

### A-3 Plasmid construction

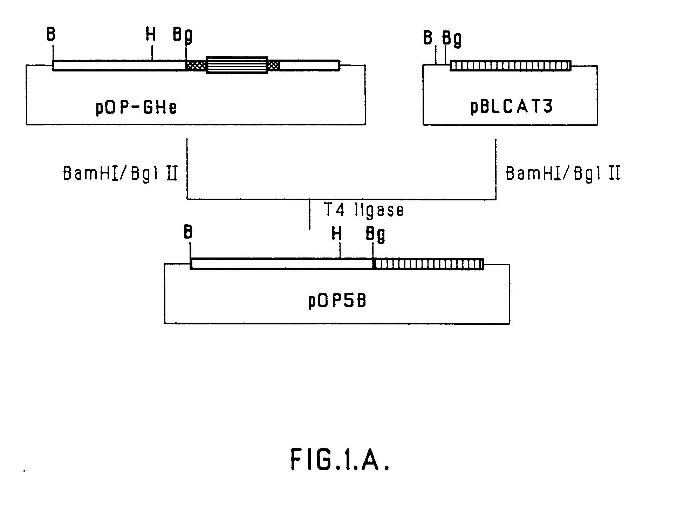

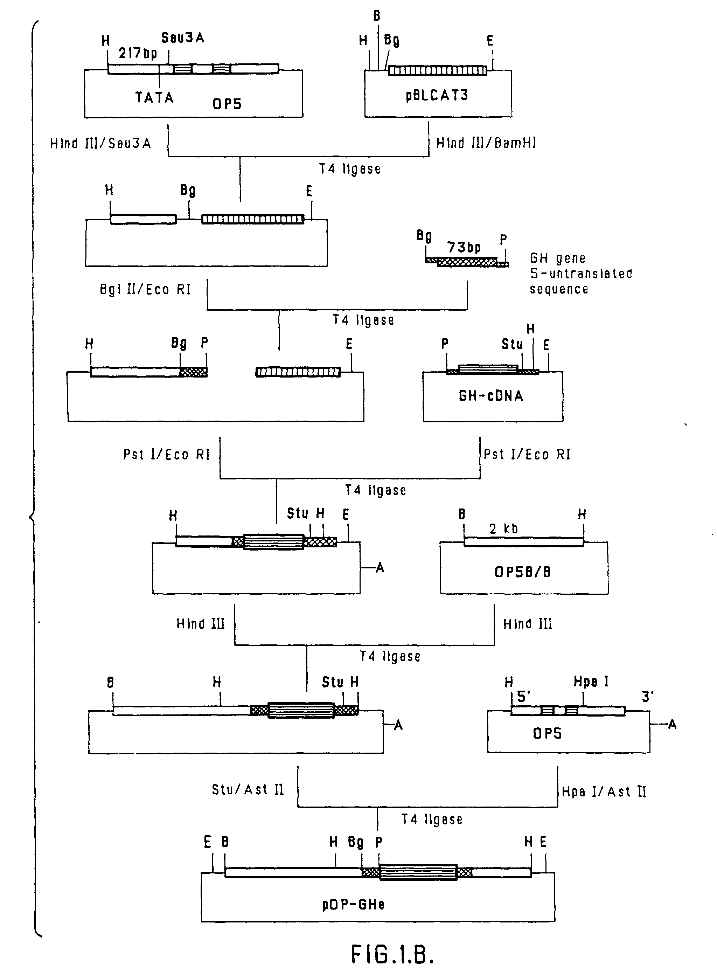

a. Ocean pout antifreeze promoter-CAT fusion gene (pOP-CAT).
   The 2 kb Bam H1-Bg1 II fragment containing the OP-AFP promoter was subcloned into plasmid pBLCAT3 (Luckow and Schutz 1987) at Bam HI, Bgl II sites to form OP-CAT fusion gene (Fig. 1A). Supercoil plasmids for transfection were prepared by ethidium bromide CsCl gradient centrifugation. The 2 kb Bam H1 - Bgl II fragment is isolated from the gene sequence of the ocean pout AFP gene as described in Hew et al (1988). The restriction map of plasmid Op5 is shown in Table 1. The construction and promoter activities of other antifreeze promoters from other fish are also included in Table II.
b. Ocean pout antifreeze promoter-salmon growth hormone fusion gene (pOP-GHe).
   The 217 bp Hind III-Sau 3A fragment from plasmid OP5 containing the OP-AFP promoter (Hew et al. 1988) of Table I, was subcloned into plasmid pBLCAT3 in pUC 18 (Luckow and Schutz 1987) at Hind III, Bam HI sites as illustrated in Fig. 1B. The plasmid was digested with BglII, and then ligated with a 73 bp BglII-Pst I synthetic linker which contains the 5'-untranslated sequence of chinook salmon GH gene (Du and Hew unpublished). The GH gene has been characterized in Hew et al (1989) and is specifically outlined in the salmon growth hormone sequences of Table III. The ligated DNA was digested with Pst I and EcoRI, and the larger fragment containing the OP-AFP promoter, chinook salmon GH 5'-untranslated sequences and pUC 18 sequence was purified by gel elution. This larger fragment was then ligated with a 709 bp Pst I-EcoRI fragment containing chinook salmon GH coding sequence and part of 5' and 3'-untranslated region (Hew et el. 1989). The resultant plasmid was cut with Hind III and cloned into a plasmid which contained the 2kb Bam H1-Hind III flanking sequence from the ocean pout antifreeze gene promoter (Hew et al. 1988). This plasmid was then digested with Stu I and Aat II, and the larger fragment which contained the OP-AFP promoter and GH coding region and part of the GH 3'-untranslated sequence, was then ligated to a 1 kb Hpa I-Aat II fragment from OP5 plasmid which included the OP- AFP gene polyadenylation and the transcription termination signals (Hew et al. 1988). Subsequent DNA sequence determination showed that the 1 kb HpaI-AatII fragment is 1.6 kb in size. It contains the 1.1 kb HpaI-HindIII fragment during the OP5 plasmid and 0.5 kb HindIII-AaI fragment from the pUC plasmid. The final construct was designated as pOP-GHe (Fig. 1B).

### A-4 Transient CAT assay in salmon cell lines

RTH-149, a rainbow trout hepatoma cell line was kindly provided by Dr. L. Gedamu. The cells were maintained at 18°C in minimus essential medium supplemented with 25 mM HEPES buffer (Gibco). The fish cells were transfected with DNA by calcium phosphate co-precipitation with glycerol shock and CAT assay were carried out essentially according to Davies et al. (1986) and modified for fish cells by Zafarullah et al. (1988).

### A-5 Transient CAT assay in Japanese Medaka.

The Supercoiled pOP-CAT plasmid DNA (approximately 500 pl, 10s copies) was microinjected into the cytoplasm of the fertilized medaka eggs prior to or immediately after cleavage. Phenol red was added to the DNA to a final concentration of 0.25% to aid in visualization of injection. CAT assays were performed according to Chong and Vielkind (1989). For 5 day embryos, batches of five embryos were used for CAT assay. For hatched fish, individual fry was used for CAT assay.

### A-6 Gene transfer in Atlantic salmon by microinjection.

The 4 kb insert in pOP-GHe was excised by EcoRI digestion and dissolved in saline buffer at a concentration of 3 *µ*g/ml. Approximately (2-3 nl, 10⁶ copy) of the DNA insert was injected through the micropyle into a fertilized, nonactivated salmon egg cytoplasm (Fletcher et al. 1988) in accordance with the procedure of the aforementioned United States patent application S.N. 278,463. Approximately 500 eggs were injected. The survival rate was 80 % as compared to the noninjected control.

### A-7 Synthesis of oligonucleotide primers for PCR

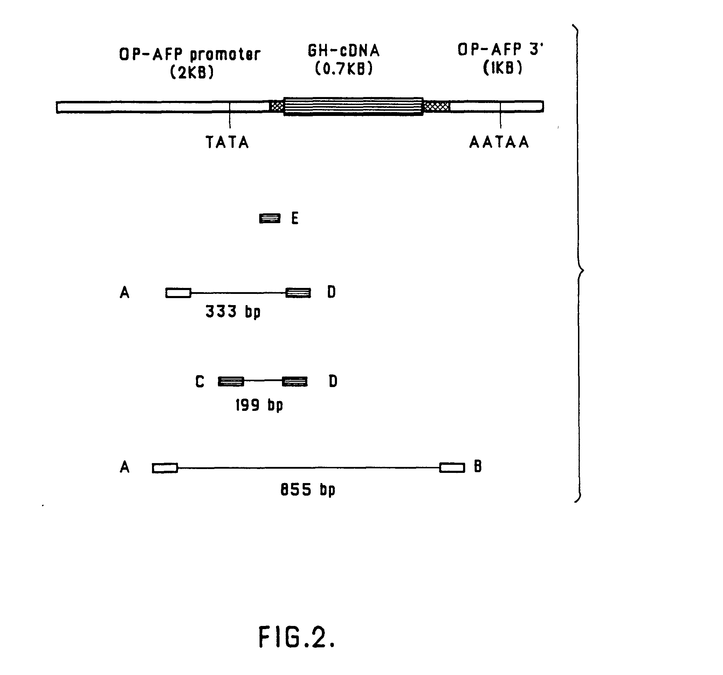

For PCR analysis, four primers were synthesized by the Biotech Service Centre, Hospital for Sick Children, Toronto, Canada. As shown in Figure 2, primer A, located at position +27 to +47 relative to the TATA box, is from the sense strand of the OP-AFP gene promoter; Primer B, located at position +861 to +881 relative to the TATA box, is from the antisense strand of the OP-AFP gene 3, flanking region. Primer C, located at position +161 to +181, is from the sense strand of GH coding sequence; While primer D, located at position +339 to +359, is from the antisense strand of GH gene.
Primer A + 27 5' -GTCAGAAGTCTCAGCTACAGC- 3' + 47 sense strand
Primer B + 861 5' -ATCTCAACAGTCTCCACAGGT- 3' + 881 antisense strand
Primer C + 161 5' -TCTGCTGATGCCAGTCTTACT- 3' + 181 sense strand
Primer D + 339 5' -ACAGAAGTCCAGCAGGAATAT- 3' + 359 antisense strand

### A-8 DNA isolation from blood cells for PCR

Thirty microliters of blood was collected from one year old fish. One microliter of the blood was lysed in 50 *µ*l of 10 mM NaOH, boiled in water bath for 3 min, then centrifuged for 3 min. Two microliters of the supernatant were used for PCR directly.

### A-9 PCR amplification

PCR was carried out in 70 *µ*l reaction solution containing 50 mM KCl, 10 mM Tris, 2.5 mM MgCl:, 1 *µ*M of each primer, four deoxyribonucleotide triphosphate at 200 *µ*M each, and 2.5 units of Taq DNA polymerase (Promega), 100 *µ*l of mineral oil was added to prevent condensation. Amplification was started by denaturating the DNA at 92°C for 3 min, followed by 30 PCR cycles. Each cycle included 1 min at 92°C (denaturation), 1 min at 60°C (annealing), and 2 min at 72°C (extension). After the final cycle, the reaction was held for another 10 min at 72°C in order to complete all the reaction. PCR was carried out by using the PTC-100 Programmable Thermal Controller (MJ Research, Inc. Ocala, FL).

### A-10 Analysis of the PCR product by agarose gel electrophoresis and southern blot

Twenty microliters of the amplified product was subjected to electrophoresis on a 0.8% agarose gel, and the DNA products were visualized by ethidium bromide staining. The DNA was transferred to a Nylon membrane (Amersham) for Southern analysis. A 17 bp GH specific oligonucleotide probe E, 5' -GAAAATGTTCAATGACT- 3', from sequence of chinook salmon GH cDNA sense strand (+277 to +294) (Fig. 2) was end labelled with p³² by T4 kinase and used as probe for Southern blot.

### EXPERIMENTAL PROTOCOL - SECOND SERIES OF EMBODIMENTS OF THE INVENTION

### B-1 Alternative embodiment for construction of ocean Pout antifreeze promoter-CAT fusion gene (opAFP-CAT)

The 2.1 kb BamHl-Bglll fragment containing the opAFP gene promoter was excised from plasmid opAFP-GHc2 (Du et al., unpublished), it was then cloned into plasmid pBLCAT3 (Luckow and Schutz, 1987) at the Bam HI and BglII sites to form the opAFP-CAT fusion gene.

### B-2 Alternative construction of an "all fish" expression cassette opAFP-V.

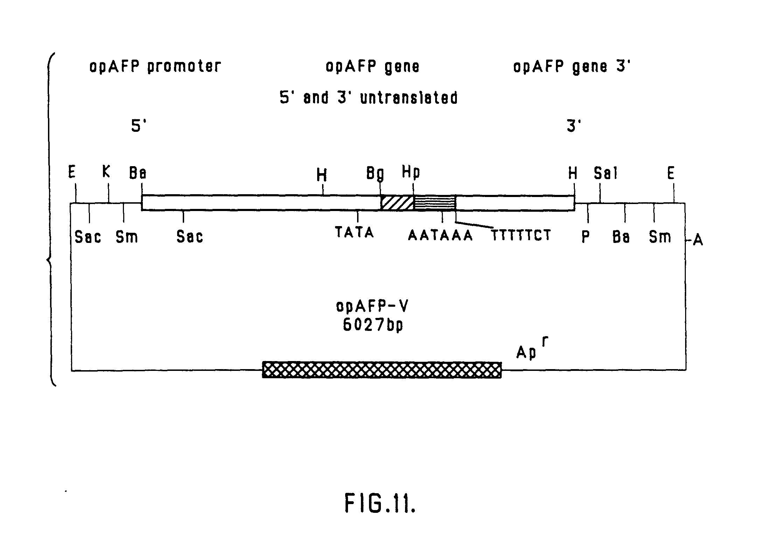

Plasmid opAFP-GHc2, derived from opAFP-GHc by replacing the 73 bp GH gene 5'-untranslated sequence with a 72 bp opAFP gene 5'-untranslated sequence, (Du et al., unpublished) was digested with Pst 1, then blunted with T4 DNA polymerase and dephosphorylated with calf intestinal alkaline phosphatase. It was then ligated to a phosphorylated 8 bp HpaI linker (5' CGTTAACG 3'). The resulting plasmid which contains the 2.1 kb opAFP gene promoter, the 63 bp opAFP gene 5'-untranslated sequence and the plasmid pUC was digested with HpaI and SaiI, and then ligated to the 1.2 kb HpaI-SalI fragment from OP-5 (Hew et al., 1988) which contains the opAFP gene 3'-sequence. The final construct was designated as vector opAFP-V. The DNA sequence starting Ba and extending to the second HindIII site H, in Fig. 11, contains 3350 bp, as in Table V.

### B-3 Transient CAT assay in salmon cell lines

RTH- 149, a rainbow trout hepatoma cell line and CHSE-214, a chinook salmon embryonic cell line, were kindly provided by Dr. L. Gedamu. University of Calgary, Canada. The cells were maintained at 18 oC in minimum essential medium supplemented with 25 mM HEPES buffer (Gibco). The fish cells were transfected with DNA by calcium phosphate co-precipitation with glycerol shock. CAT assay were carried out essentially according to Davies et al. (1986) as modified for fish cells by Zafarullah et al. (1988).

### B-4 Transient CAT assays in Japanese medaka

Japanese medaka (Oryzias latipes) were maintained at Dr. J. Vielkind's laboratory, Cancer Research Centre, Vancouver. The reproductive activities of the adults were induced by artificial photoperiod according to the method reported by Chong and Vielkind (1989). Fertilized eggs attached to females were collected 1-2 h after the onset of light and maintained in Ringer solution (0.75% NaCl, 0.02% KCl, 0.02% CaCl₂, pH 7.3) at 12°C prior to injection. Injected medaka embryos were reared in medium containing 0.1% NaCl, 0.003% KC1, 0.004% CaCl₂.2H₂O, 0.016% MgSO₄.7H₂O, 0.0001% methylene blue and transferred to aquarium water immediately after hatching.

Supercoiled opAFP-CAT plasmid DNA (approximately 500 pl, 10⁶ copies) was microinjected into the cytoplasm of fertilized medaka eggs at the 1 or 2 cell stages. Phenol red was added to the DNA to a final concentration of 0.25% to aid visualization during injection. CAT assay were performed according to Chong and Vielkind (1989). Individual embryos were used for each CAT assay.

### B-5 DNA sequencing

The dideoxy chain terminator sequencing method was used to sequence opAFP-V. A series of clones containing DNA fragments of different lengths were generated by EXO-3 nuclease deletion (Promega Erase-a-Base deletion kit). Double stranded DNA purified from these clones were used as templates for DNA sequencing (Phamacia sequencing kit).

### TEST RESULTS OF THE ABOVE PROCEDURES - ACCORDING TO THE FIRST SERIES OF EMBODIMENTS

### 1. Ocean pout AFP promoter can function in salmonid cells in vitro.

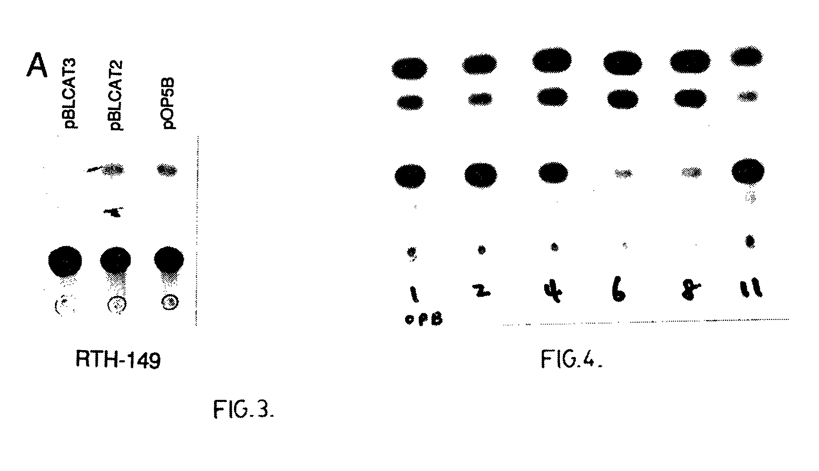

To test the effectiveness of the OP-AFP promoter, the OP-CAT construct was transfected into RTH 149 cell line for CAT assay. As showed in Figure 3, CAT activity was clearly detected. The level of CAT activity resulted from OP-CAT was comparable to that from pBLCAT2, which has the thymidine kinase promoter from Herpes simplex virus; however, when these cells transfected with pBLCAT3, a promoterless CAT construct, little or no CAT activity was detected. Similar results were obtained with Chinook salmon embryonic cells (CHSE-124) and Chum salmon heart cells (CHH-1). These results suggest that OP-AFP promoter can be used to target the GH gene expression in salmonids. Although the salmonids including the rainbow trout lack the AFP gene, these cells contain all the transcription factors required for the expression of the AFP gene.

### 2. Ocean pout AFP promoter can function in Japanese Medaka in vivo.

To further investigate the suitability of the OP-AFP promoter in gene transfer, the OP-CAT construct was microinjected into medaka eggs. CAT activity was determined from embryos at different times during development. As showed in Figure 4, the CAT activity was first detected at 48 hours after the injection, the activity reached the maximum at 6-7 days, then the CAT activity began to decline. However the CAT activity was still detectable even in the hatched fish (11-12 days). In contrast, the CAT activity was not detected in the uninjected embryo or embryo injected with pBLCAT3. This result confirms that the OP-AFP promoter is active in a variety of fish species.

### 3. The PCR-based screening strategy.

To screen for the presence of transgenic salmon, three different sets of primers were used, Primers A/D, primers C/D and primers A/B (Fig. 2). The basis for using primer A/B is that the sequences of primer A and Primer B are specific for OP-AFP gene, which are absent in Atlantic salmon, therefore DNA from the nontransgenic salmon can not be amplified when using primers A/B for PCR. Only the DNA from the transgenic fish can be amplified by using primer A/B, and will generate a 855 bp DNA fragment by PCR.

The basis of using primers A/D is similar as using primer A/B. Although primer D is derived from chinook salmon GH cDNA, and might hybridize with the endogenous Atlantic salmon GH gene, primer A is specific for the OP-AFP gene. Hence the DNA from nontransgenic fish can not be amplified by using primers A/D, only the DNA from transgenic fish can be amplified and generate a 333 bp DNA fragment.

The basis for using primers C/D is different from that of other two sets. The sequences for both primer C and D are from the chinook salmon GH cDNA, which could hybridize with the Atlantic salmon GH gene, and therefore DNA from Atlantic salmon can be amplified by using primer C/D. However there is an intron (intron 2) between primer C and primer D, the distance between primer C and primer D is 344 bp (Du and Hew unpublished). Primers C/D will generate a 344 bp fragment in all the DNA samples. The transgene pOP-GHe was constructed using chinook salmon GH cDNA which lacks the intron and the distance between primer C and primer D is 199 bp. Primers C/D will generate two fragments in transgenic salmon, a 344 bp from the endogenous GH gene and a 199 bp from the GH cDNA insert.

### 4. The identification of transgenic salmon by PCR.

Preliminary analysis of the DNA extracted from 100 one month old salmon embryos revealed that two of them (2%) contained the injected sequence (pOP-GHe).

Eight months after hatching, the salmon were large enough to tag for identification. At this time, 50 of the approximately 500 salmon in one aquarium were weighed and blood sampled for PCR analyses. These 50 included the 14 largest salmon in the aquaria (>8 gm body weight) and 36 additional fish with body weights ranging from 5 to 14 gm.

The PCR analysis was carried out without the analyst knowing the size of any of the fish. In other words, the analysis was carried out "blind" in order to be certain to eliminate any bias by the analyst.

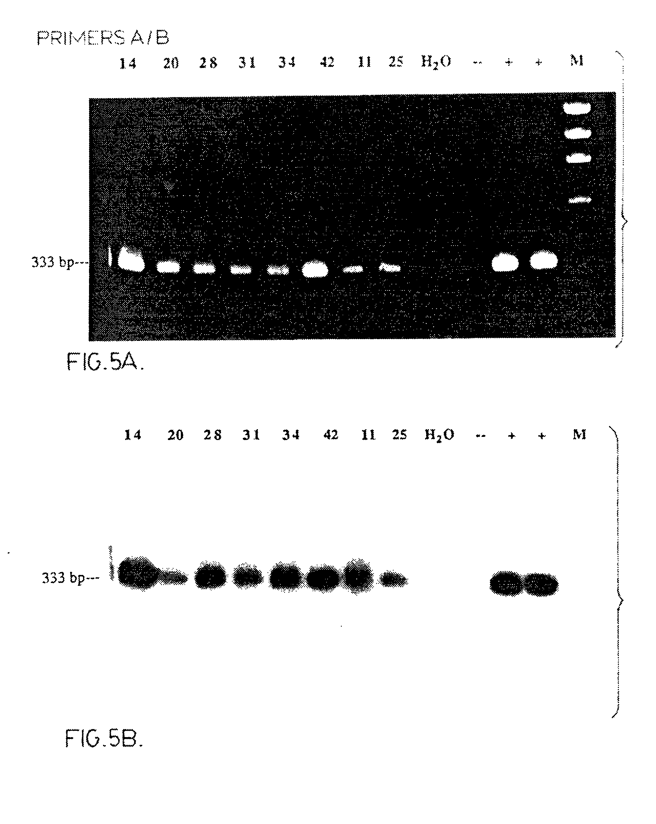

Eight month old Atlantic salmon developed from the eggs injected in November 1989 with pOP-GHe were bled in October 1990. The DNA from the nucleated blood cells were used directly for PCR analysis using primer A/D to determine the presence of the pOP-GHe transgene. Out of 50 fish analyzed, eight were shown to be positive. As showed in Fig. 5A, a 333 bp fragment was generated from salmon #14, #20, #28, #31, #34, #42, #11, #25 and the positive control (pOP-GHe), in contrast this 333 bp fragment was absent in the noninjected salmon. The size of the amplified fragment (333 bp) corresponded with the size predicted from the transgene sequence.

To confirm that the amplified 333 bp DNA fragment was derived from the transgene pOP-GHe, the DNA was transferred to a nylon membrane for Southern blot hybridized with a GH specific probe E (Fig. 2) which was from the sequence between the primer A and primer D, and the result showed that all the 333 bp hybridized with the probe E (Fig. 5B). This confirmed that the 333 bp DNA fragment was in fact derived from the transgene pOP-GHe.

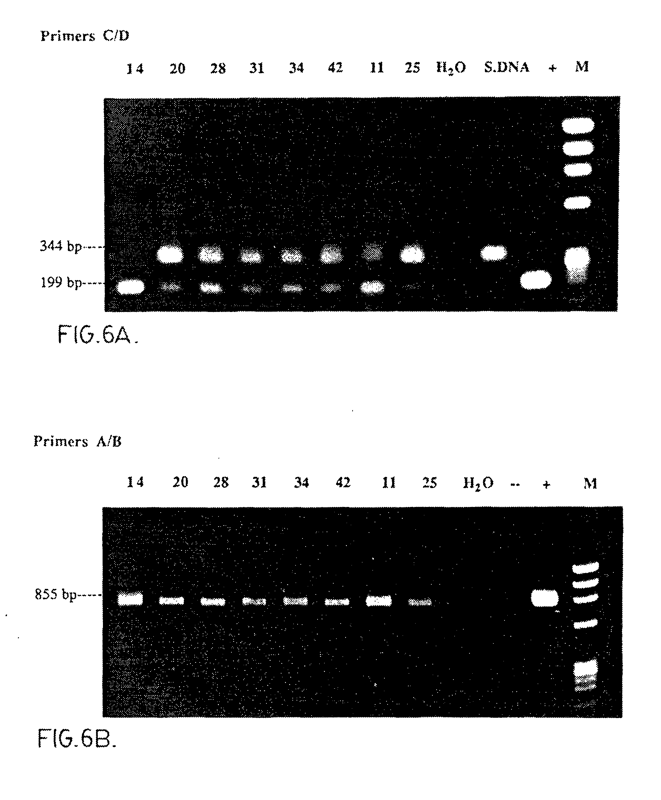

To further confirm that the presence of transgene pOP-GHe in the positives, the DNA were amplified by using primers C/D. As discussed in the screening strategy, the expected 344 bp DNA fragment derived from the endogenous Atlantic salmon GH gene were found in all the salmon analyzed. An additional smaller DNA fragment (199 bp) were found from fish #14, #20, #28, #31, #34 and #42, #11 and #25 (Fig. 6A). This 119 bp fragment was derived from the chinook salmon GH transgene. These positives were the same ones as obtained by using primers A/D, thus confirming that fish #14, #20, #28, #31, #34, #42, #11 and #25 were transgenic salmons.

### 5. The GH coding regions of the transgenes are intact in the transgenic salmon.

To determine the integrity of the GH coding sequence of the transgene, primers A/B were used for PCR analysis. Primers A and primer B were derived from the sequences of the OP-AFP gene 5' and 3', which were located outside of the 5' and 3' of the GH coding sequence in the transgene (Fig.2), therefore, if the GH coding in the transgene was intact in the transgenic fish, a 855 bp DNA fragment should be generated when using primers A/B to amplify the DNA from the transgenic fish. As showed in Figure 6B, a 855 bp fragment was found in all the eight positives, indicating that the GH transgene was intact in the transgenic salmon.

### 6. The growth performance of the transgenic fish.

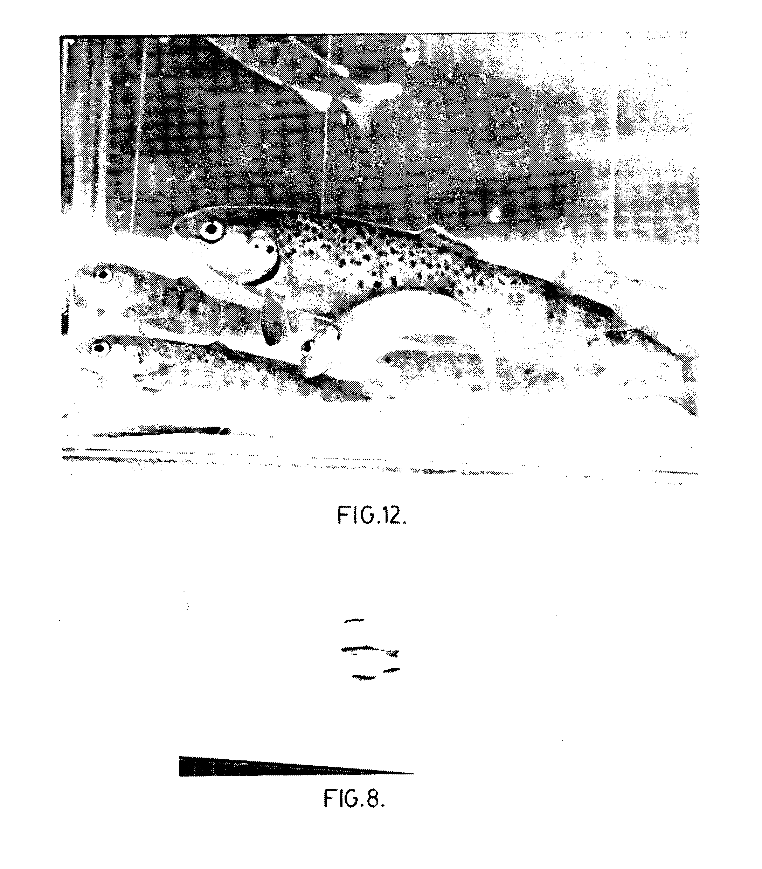

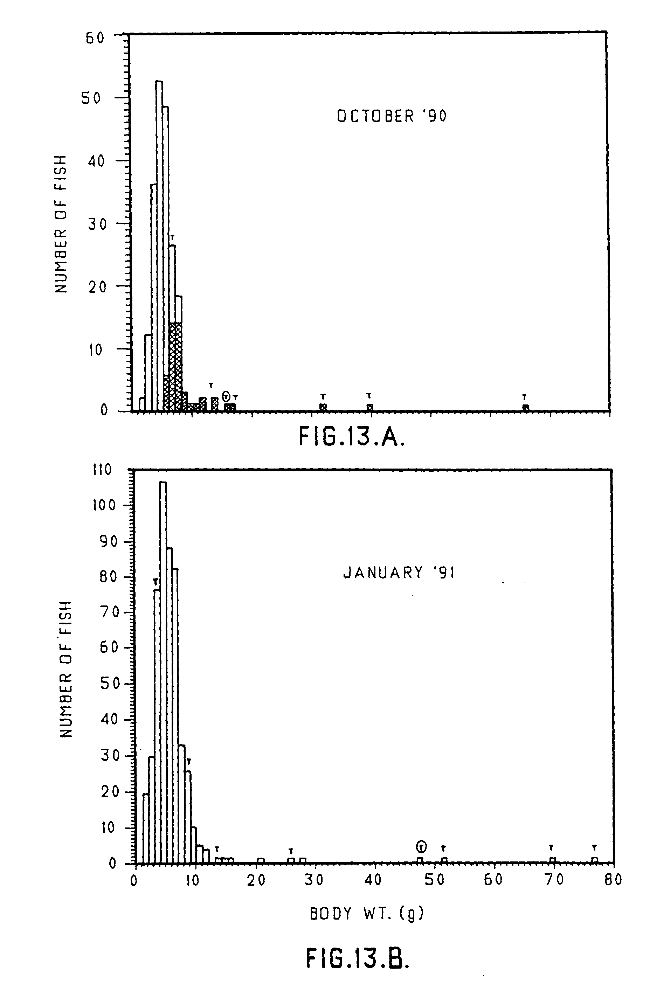

a. Weight of the transgenic fish
   PCR analysis showed that eight fish were transgenic. Two fish (#11, #25) died in July 1990. Therefore, only the six remaining transgenic fish were analyzed.
   Of the six salmon found to be transgenic for the growth hormone gene construct, five of them were amongst the six largest fish in the aquarium. The chances of the observation occurring by coincidence are exceedingly low. The average weight of the six transgenic fish was 29.2 ± 0.3 gm. The average weight of all 459 fish in the aquarium was 5.06 ± 1.0 gm. Thus on average the transgenic fish were four times larger than the non-transgenic controls and approximately six times the average size of the salmon in the aquarium. The largest transgenic fish in the aquarium was eight times larger than the non-transgenic controls.
b. The growth rate of the transgenic fish
   In order to estimate growth rates, the fifty fish that had been blood sampled and tagged were reweighed 100 days later. The mean growth rates of the six transgenic salmon during this period was 0.766 ± 0.18% per day, while the 24 non-transgenic fish grew at 0.224 ± 0.03% per day. This difference is statistically significant.
c. Weights of the transgenic fish
   The body weights of the two transgenic salmon that had died in July 1990, numbers 11 and 25, were 8.07 g and 12.1 g respectively. These salmon were considerably larger than all other salmon in the aquarium at that time. The mean body weight of 10 fish selected at random from the approximately 500 fish in the aquarium was 1.4 ± 0.17 g.
   The size frequency distribution of all 200 salmon that were weighed in October 1990, including the fifty that were bled and tagged is presented in Figure 13A. Of the seven transgenic salmon found amongst the fifty fish sampled, six were the biggest fish in the aquarium (Figure 1#A). The mean body weight of the 200 fish was 5.91 ± 0.43 g. The body weights of the three largest transgenic salmon were greater than two standard deviations larger than this mean. The mean body weight of the seven transgenics was 27.3 ± 7.8 g, while the forty-three non-transgenic siblings weighed 7.4 ± 0.26 g. Thus on average the transgenics were 3.7 times larger than their non-transgenic siblings. The largest transgenic salmon (#28, 65.8 g) was 8.9 times the mean weight of the controls. The three largest transgenic salmon had lost all evidence of parr markings and had taken on the silvery appearance of smolts (Figure 12). Growth hormone has been implicated in this process.
   The size frequency distribution of all 494 fish present in January 1991, and the weights of the individual transgenics are presented in Figure 12B. The largest transgenic salmon sampled in October 1990 (#28) died following blood sampling, and is not present in the Figure.
   The average body weight of all (484) salmon in the aquarium was 6.36 ± 0.26 g. The average body weight of the non-transgenic salmon (476 fish) was 5.94 ± 0.14 g, while the mean weight of the transgenics was 37.0 ± 10.2 g; approximately six times larger than the non-transgenics. Five of the eight transgenics in this group has body weights exceeding two standard deviations of the mean value for the aquarium. The body weights of two of the 476 non-transgenic salmon also exceeded two standard deviations of the aquarium mean. The largest transgenic salmon in January 1991 (#31, 76.7 g) was approximately 13 times the average weight of the controls.
   The growth rates of the transgenic salmon (6) and their non-transgenic siblings (43) from October 1990 to January 1991 are presented in Table VI. It is evident that on average the transgenic salmon were significantly larger and grew significantly faster in weight and length, than non-transgenic fish reared in the same aquarium. In addition, the condition factors of the transgenics were slightly, but significantly lower than those of the controls. Increases in weight of the transgenic fish ranged from 0.48 to 1.6% per day. Only two of the 43 non-transgenic controls had growth rates exceeding those of the slowest growing transgenic.
   Smolting is a very complicated transformation process involving many morphological and physiological changes (see review by Hoar 1988). One of the morphological changes is the silvering of smolt. In our studies, the transgenic salmon silvers earlier than the control, suggesting that transgenic salmon smolts earlier (Fig.8). It has been reported that the fish size and growth rate appear to be the significant factors in controlling the salmon smolting (see review by Hoar 1988), this is supported by our observation that the transgenic salmon smolt earlier than their control. Therefore, the transgenic salmon appears to be a good model for the study of salmon smolting.

### TEST RESULTS OF THE ABOVE PROCEDURES ACCORDING TO THE SECOND SERIES OF EMBODIMENTS

### 1. The ocean pout AFP promoter can function in salmonid cells in vitro.

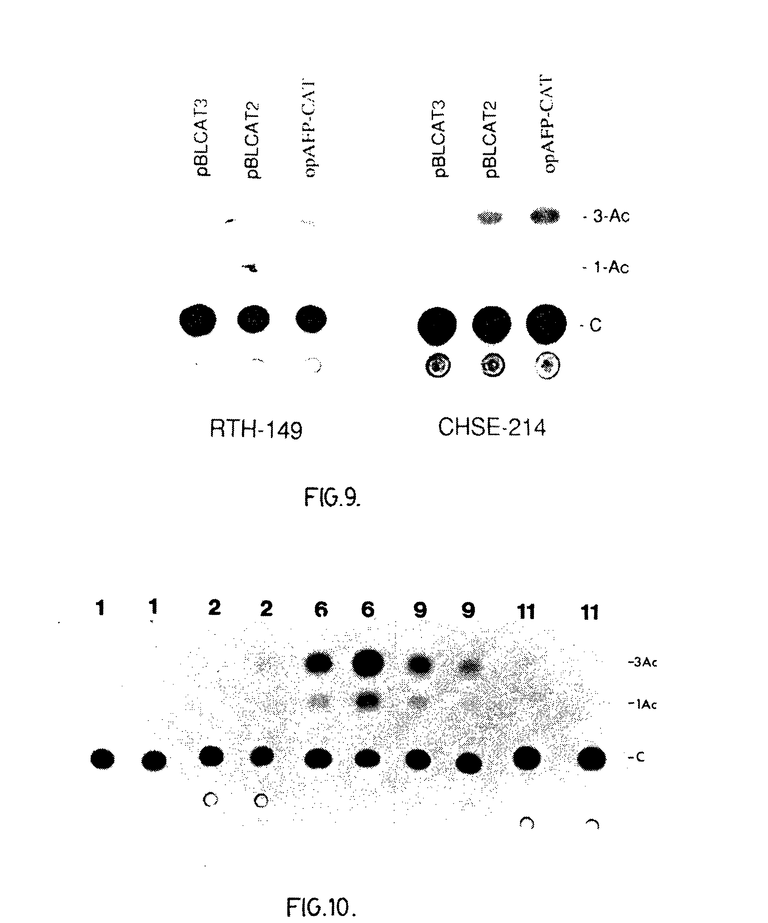

CAT assays were used to test the effectiveness of the opAFP promoter. The opAFP-CAT construct was transfected into two salmonid cell lines, RTH 149, a rainbow trout hepatoma cell line and CHSE-214, a chinook salmon embryonic cell line. As shown in Figure 9, CAT activity was clearly detected in both of them. The level of CAT activity resulting from opAFP-CAT was comparable to that from pBLCAT2, which has the thymidine kinase promoter from Herpes simplex virus. When these cells were transfected with pBLCAT3, a promoterless CAT construct, little or no CAT activity was detected. These results suggested that although the salmonid cells lack the AFP gene, these cells contain the transcription factors required for the basal expression of the AFP gene, indicating its usefulness in transgenic studies.

### 2. The ocean pout AFP gene promoter can function in Japanese medaka embryos in vivo.

To further investigate the suitability of using the opAFP gene promoter in gene transfer, the opAFP-CAT construct was microinjected into medaka embryos at 1 or 2 cell stages. CAT activity was determined from individual embryo at different times during embryonic development. As shown in Figure 10, CAT activity was first detected 2 days after injection, the activity reached a maximum at 6-8 days, then CAT activity began to decline. However CAT activity was still detectable even in the hatched fish (11 days). In contrast, little or no CAT activity was detected in non-injected embryos or embryos injected with pBLCAT3 (data not shown). These results further demonstrated that the opAFP gene promoter was active in vivo in a different species of fish lacking the AFP genes.

### 3. Design and construction of a universal gene transfer cassette, opAFP-V.

The above results from the CAT assay in salmonid cell lines and medaka embryos suggest that the opAFP promoter is active in fish which do not nominally express AFP. This is further supported by our recent GH gene transfer studies in Atlantic salmon. When the opAFP gene promoter was ligated with the salmon GH cDNA and the fusion gene microinjected into Atlantic salmon, the transgenic salmon showed a dramatic enhancement in growth rates (Du et al. 1992). Overall, these experiments indicate that the opAFP promoter is active in a variety of fish species.

To facilitate the use of the opAFP gene promoter as a useful vehicle for gene transfer studies, and to simplify the construction of other fusion genes using this promoter, an expression vector , opAFP-V, was cloned in pUC using the opAFP gene promoter, the 5'-untranslated sequence and the opAFP gene 3' sequence. The 2.1 kb promoter is required for active transcription and the 1.2 kb 3'-sequence is preferred for polyadenylation and transcription termination. As shown in Figure 11, the 2.1 kb opAFP gene promoter was linked with the 63 bp opAFP gene 5'untranslated sequence by a unique BgIII site, and the 5'-untranslated sequence was linked with its 3' sequence by a unique Hpal site. Where and how to insert a gene into this vector depends on the nature of the gene to be inserted, i. e., a cDNA or a genomic DNA, with or without its own 5'untranslated sequence.

To insert a cDNA sequence which contains a short or no 5'-untranslated region into opAFP-V, the insertion should be at the HpaI site, which is right after the opAFP gene 5'untranslated sequence. The Hpal site is the only HpaI site in the vector, thus it can be cleaved by single HpaI digestion and cDNA clones of interest without the 5'-untranslated sequence can be directly cloned into this unique Hpal site. The 5'-untranslated region from opAFP gene may serve as a leader sequence for ribosomal scanning for translation initiation.

To insert a cDNA sequence with a relatively long 5'-untranslated region, the insertion could be targeted to the unique BglII site which is before the opAFP gene 5'-untranslated sequence. To avoid having the extra opAFP gene 5'-untranslated sequence, the opAFP gene 5'-untranslated sequence can be removed by BglII/HpaI digestion. The insertion could also be at the Hpal site and generate a mRNA with a long fusion 5'-untranslated sequence.

Since genomic genes contain their own 3' sequences which function as a transcriptional terminator, the 3' opAFP gene sequence is not required for the cloning of genomic genes. The opAFP gene 3' sequence can be removed from the vector by BglII/SaII or Hpal/SaII digestion depending on the length of the 5'-untranslated sequence in the inserted genomic gene.

As shown in Figure 11, several additional unique restriction sites are present at the 5'- and 3'- ends in the vector, such as EcoRI, Ppnl, Smal and BamHI sites at the 5'-end, and the Pstl, SaII, BamHI, Smal and EcoRI at the 3'-end. These enable the excision of the intact fusion gene from the vector.

### 4. The complete DNA sequence of OpAFP-V.

To assist the analysis of the integration and expression of the transgene with this vector, the complete DNA sequence of opAFP-V was determined. The full length of the opAFP-V is 6027 bp, with 2677 bp derived from pUC sequence (Yanisch-Perron et al., 1985) and 3350 bp derived from opAFP DNA sequence. The complete opAFP DNA sequence is shown in Table V. The opAFP gene promoter spans 2.1 kb, it has a "CAAT" box and a "TATA" box located at position 2006 and 2049 respectively. Following the promoter sequence is the opAFP gene 5'-untranslated sequence, which is linked to the promoter by a synthetic BglII linker (at position 2116). The exact size of the 5'-untranslated sequence is not known because the ocean pout has 150 copies of the AFP gene (Hew et al., 1988) which have very similar but not identical gene structures, therefore it is difficult to determine the CAP site for this particular AFP gene. In eukaryotic genes a CAP site is usually located 25-30 bp downstream from the "TATA" box. Therefore, the 5'-untranslated sequence of opAFP gene is about 90 bp. The 5'-untranslated sequence present in opAFP-V is 63 bp long, which is located between the BgIII and the Hpal sites. The opAFP gene 3' sequence is linked with the promoter and the 5'-untranslated sequence by a unique HpaI site (at position 2188). The 3' sequence spans 1.2 kb, which contains an 80-90 bp 3'-untranslated sequence, and a 1.1 kb 3'flanking sequence. A poly(A) signal "AATAAA" and a potential transcription termination signal CT" are located at position 2253 and 2285 respectively.

The DNA sequence of the opAFP gene was analyzed for the presence of any liver-specific sequences. Two 17 bp fragments with 70% identity to the liver-specific promoter element HPI of the Xenopus albumin gene (Schorpp et al., 1988), were demonstrated at position 1004 to 1020 and 1944 to 1960 (Table V). These two fragments were located at approximately -1 kb and -100 bp up stream of the CAP site respectively. The Xenopus albumin HP1 sequence is suggested as the binding site for a liver-specific transcription factor LF-B1 (De Simone and Cortese, 1988). However, the function of these two presumptive liver-specific sequences remains to be investigated.

Recently, PCR has become a useful tool to analyze the DNA where the source of DNA is limited. PCR has been used in screening for transgenic mouse (Chen and Evans 1990), the blood cell were lysed by SDS and DNA was used directly for PCR. Recently, Hanley and Merile (1991) reported the transgene detection in mouse by PCR using unpurified tail DNA. Our data revealed that 1 *µ*l of blood is sufficient for screening of transgenic fish directly by PCR. This protocol has been adopted for the routine analysis in the laboratory for the detection of several thousand samples.

It is appreciated that there are other promoter systems which, in accordance with this invention, work equally in a transgenic gene construct. These include promoter sequences isolated from wolffish, winter flounder, sea raven, and other AFP or AFGP-containing fishes.

It is appreciated from the above discussion of various embodiments of the invention as they relate to the promoter, optional 5' untranslated sequence as a gene precursor and the 3' terminal sequence that there are, of course, many functional equivalents to the stated sequences, not only with respect to the particular sequence of Table V, but as well as demonstrated by the promoter sequences as isolated from other species than the ocean pout, namely the wolffish as described with respect to Table IV. The promoter sequence, according to this invention, is applicable to a variety of gene products since they are derived from fish genes and are therefore compatible with a variety of types of fish. Hence the promoter sequence is referred to as "all-fish" gene construct. In view of the advantage of the promoter sequence being liver specific or liver-predominant for expression, which is a tissue well suited for the synthesis and secretion of transgene products, there is a diversity of AFP/AFGP promoters useful in making the "all fish" gene conduct (Gong et al, 1992).

In view of the AFP promoter sequence being absent in most fish species, detection of transgenic species is readily provided by PCR or other forms of gene sequence amplification techniques, such as ligase chain reaction. Furthermore, since most fishes do not contain these AFP genes, it is easier to modify the transgene using the AFP promoter without affecting the performance of endogenous genes. Also the AFP gene sequence including both the 5' and 3' ends contains functional DNA sequences important for normal DNA transcription and termination and tissue specific expression. These DNA sequences can further be modified to improve its experience level at will and responsive to many external signals such as hormones, growth factors, etc.

Although preferred embodiments of the invention are described herein in detail, it will be understood by those skilled in the art that variations may be made thereto within the scope of the appended claims.

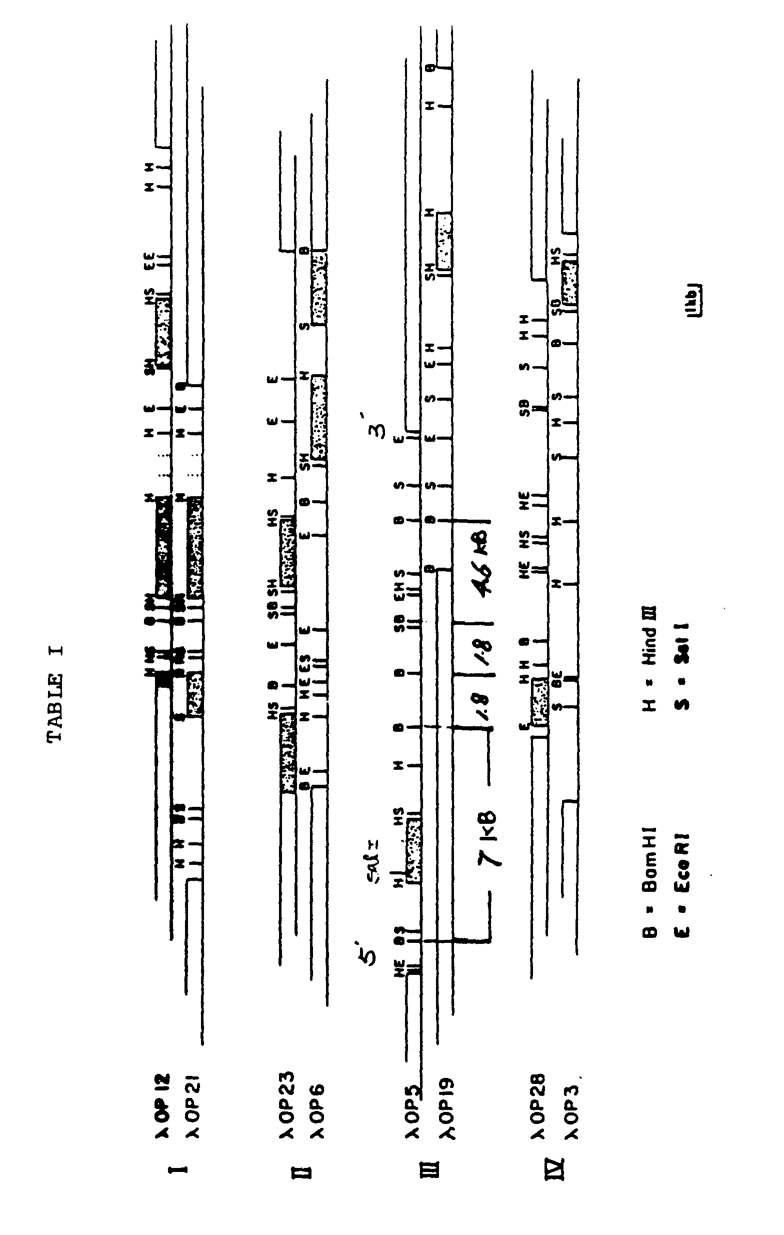

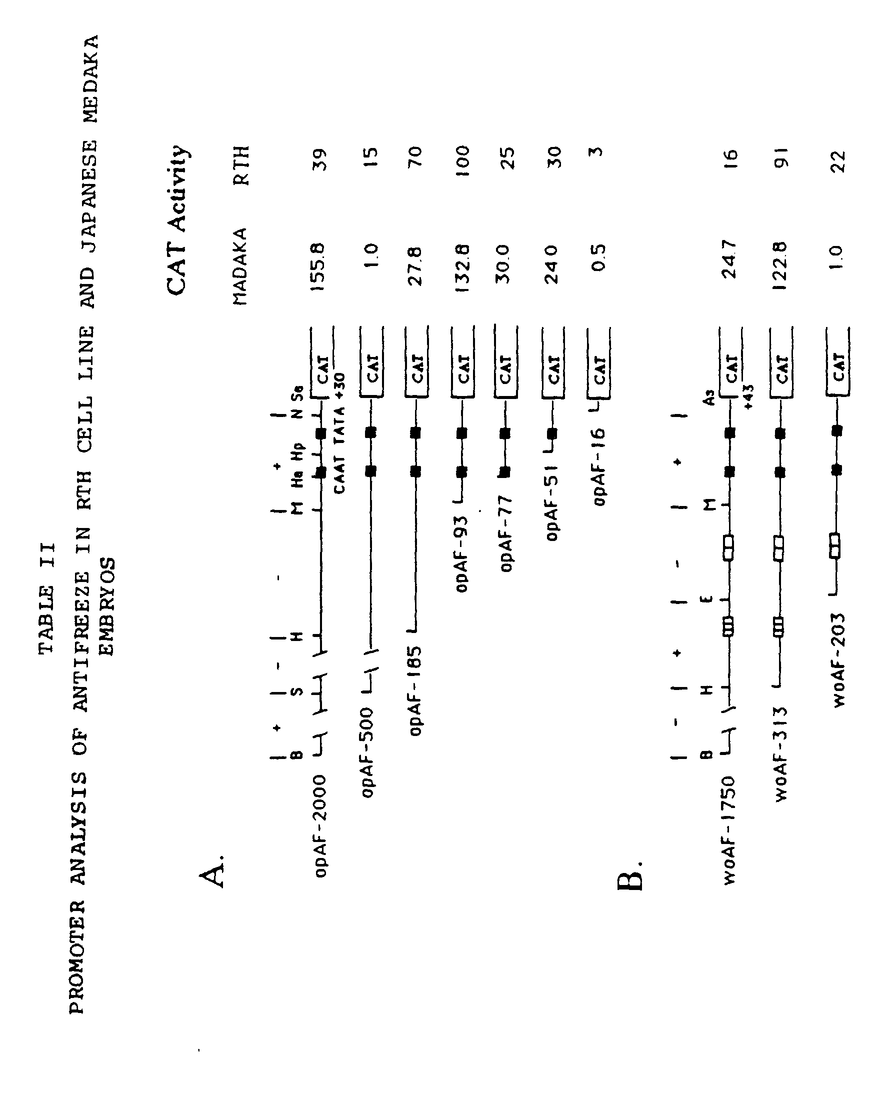

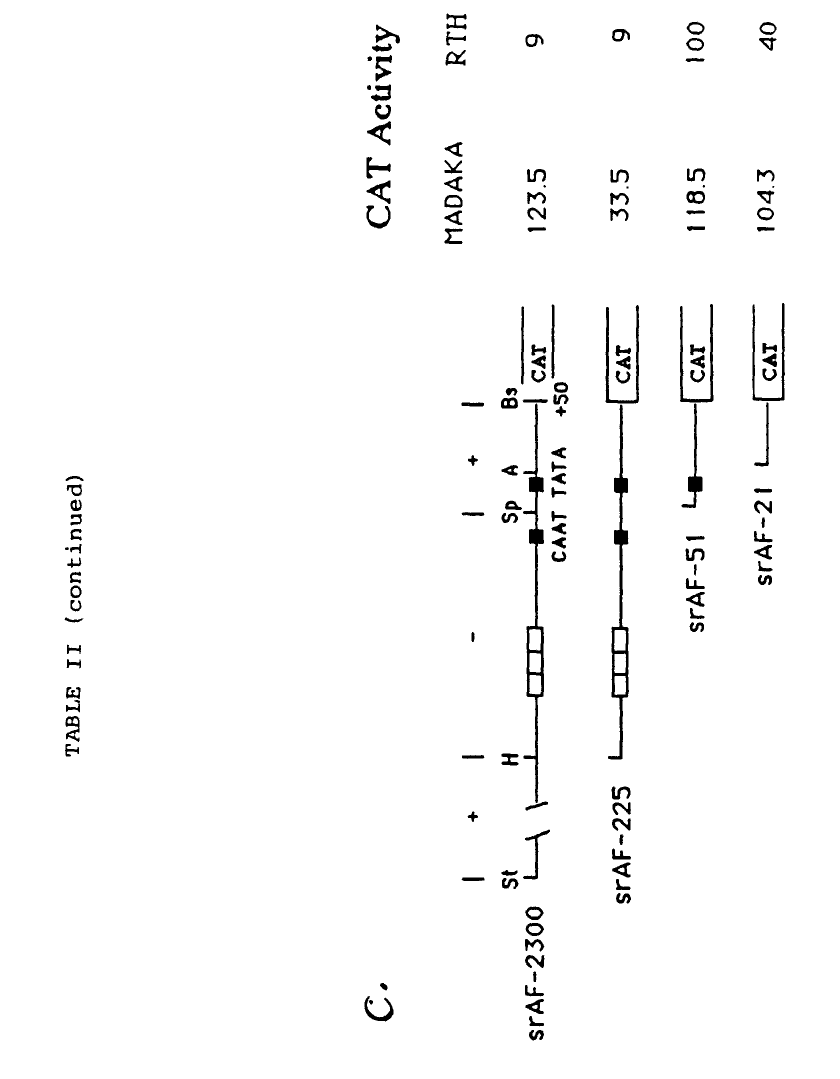

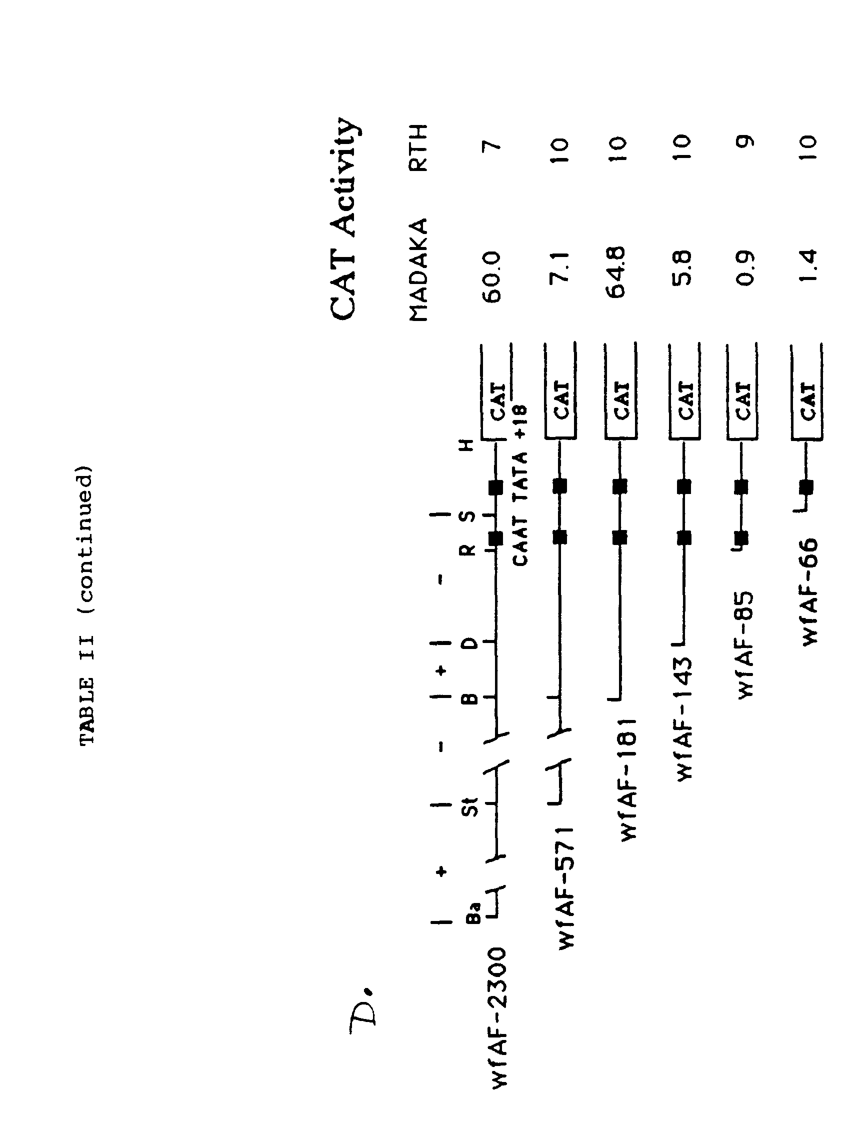

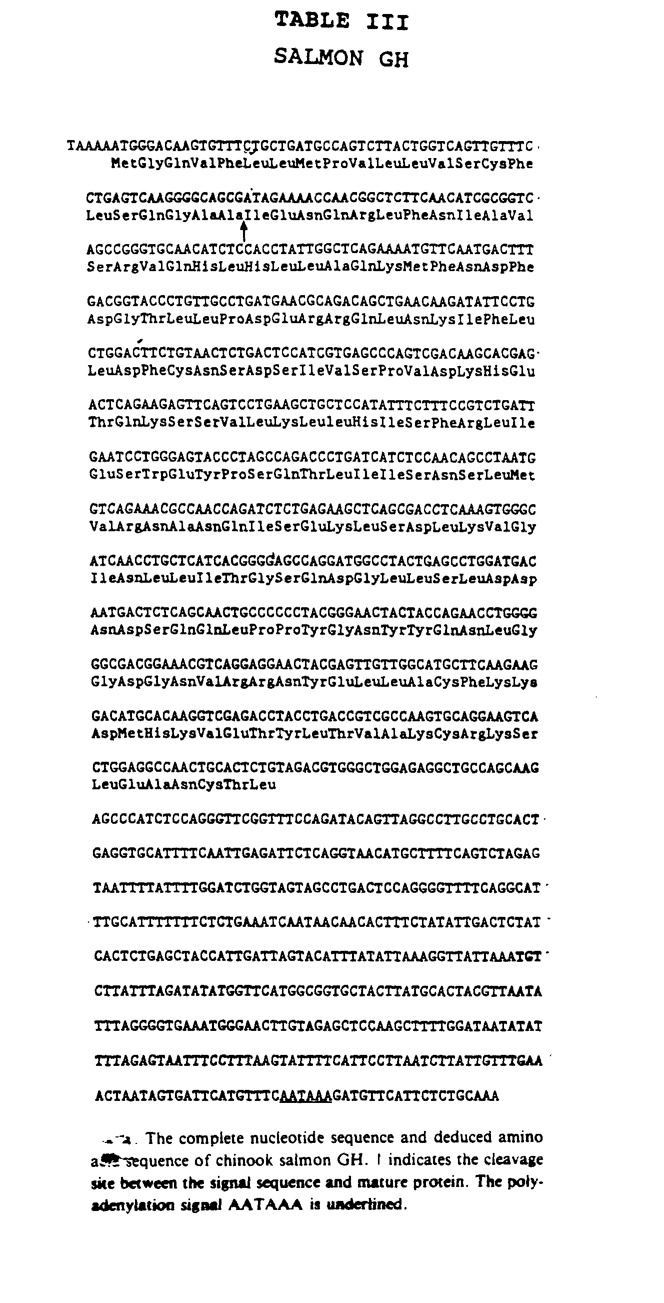

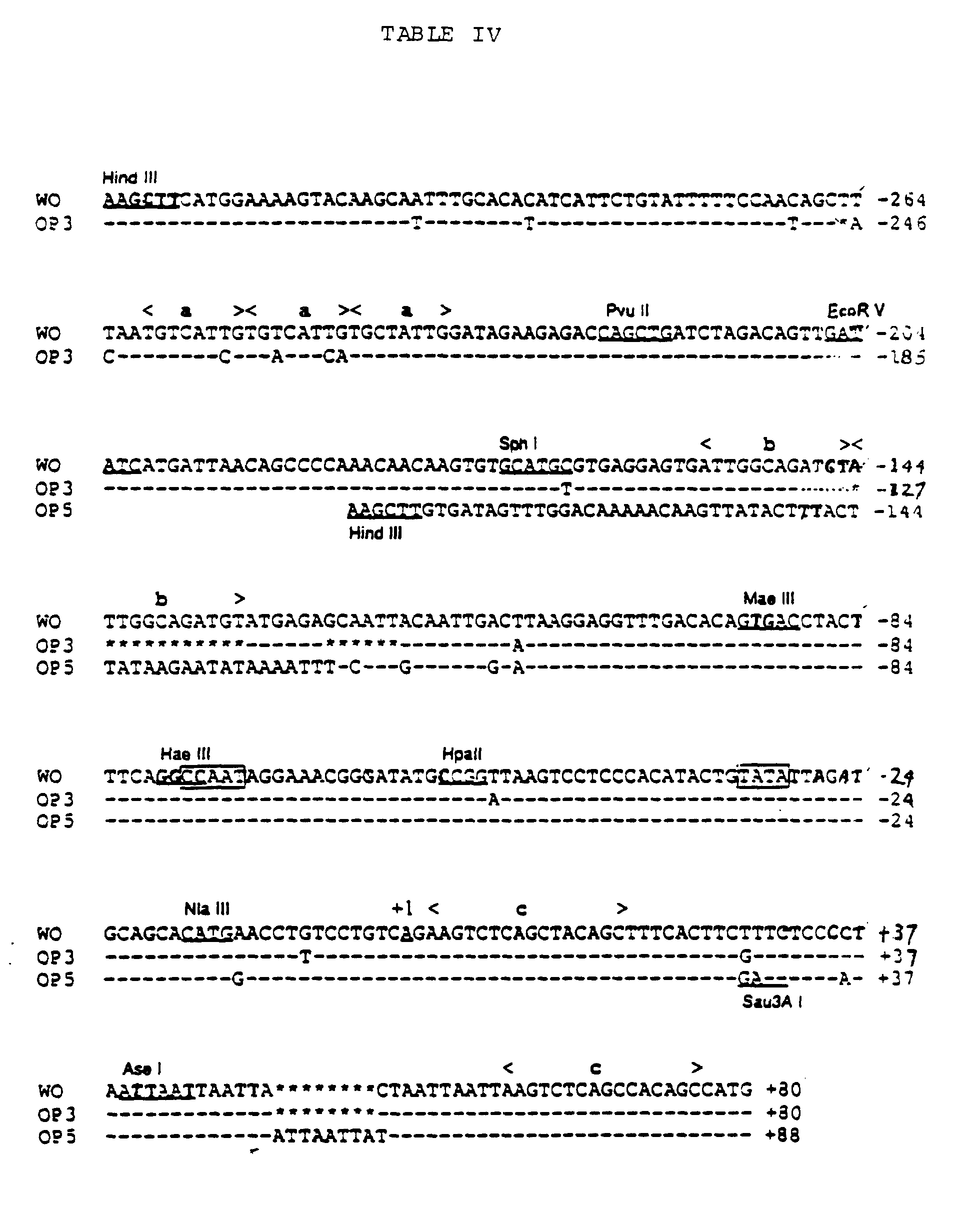

### TABLE V

The complete ocean pout DNA nucleotide sequence in opAFP-V. The "CAAT" and "TATA" sequences are boxed; the poly(A) signal (AATAAA) and the predicted transcription-termination signal (TTTTTCT) are underlined by double lines; the two presumptive liver-specific sequences are underlined by single line; and the unique BgIII and Hpal sites for gene insertion are indicated.

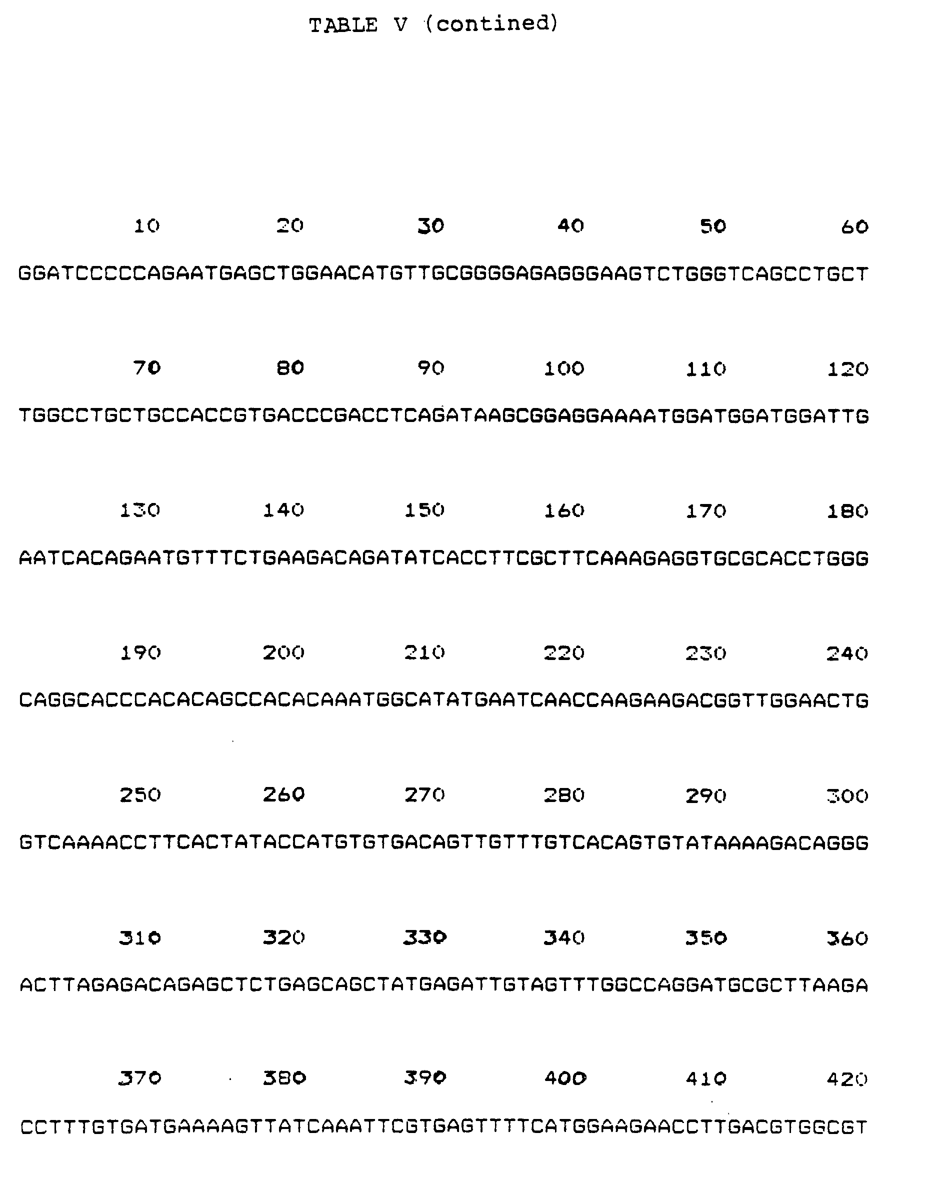

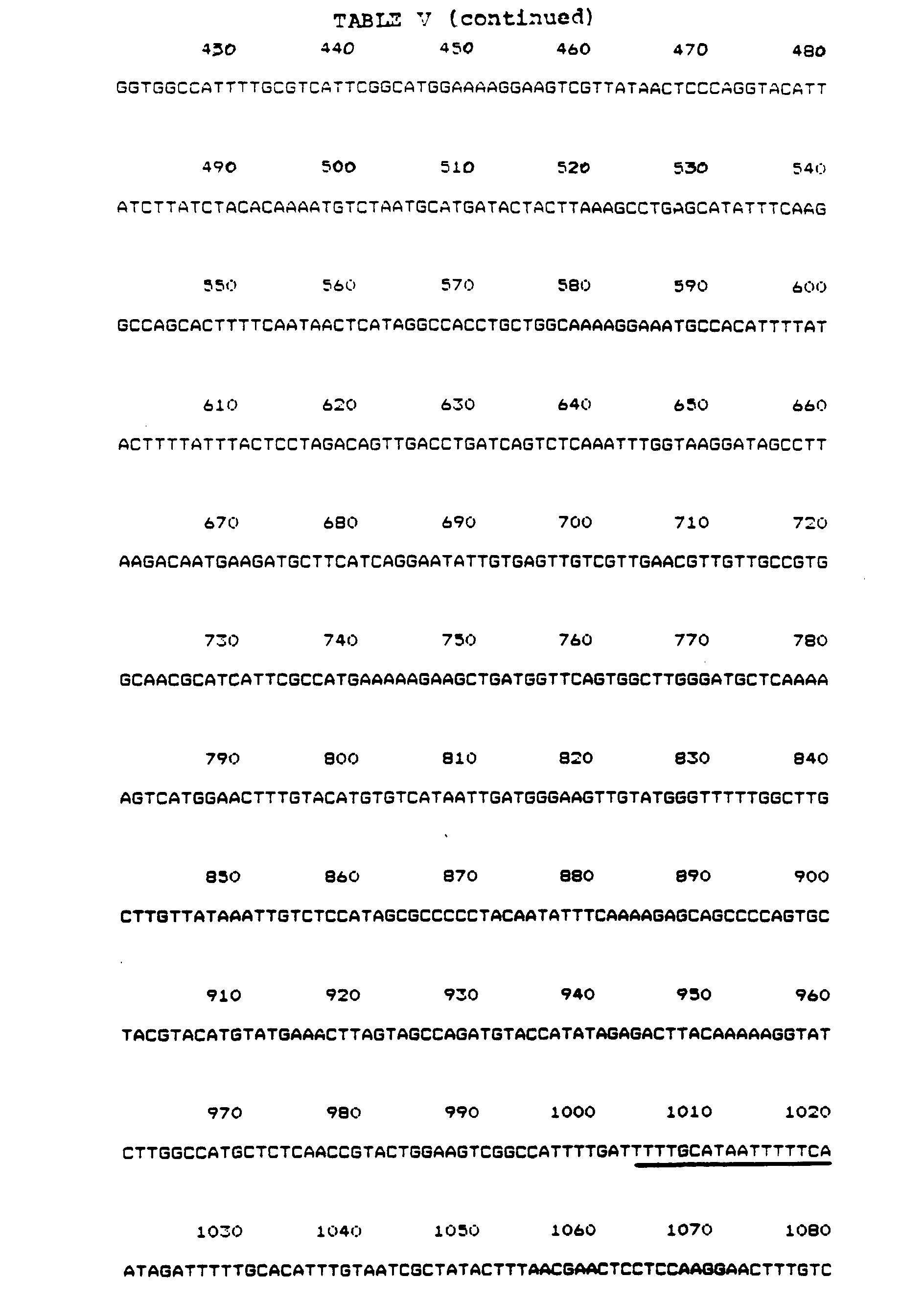

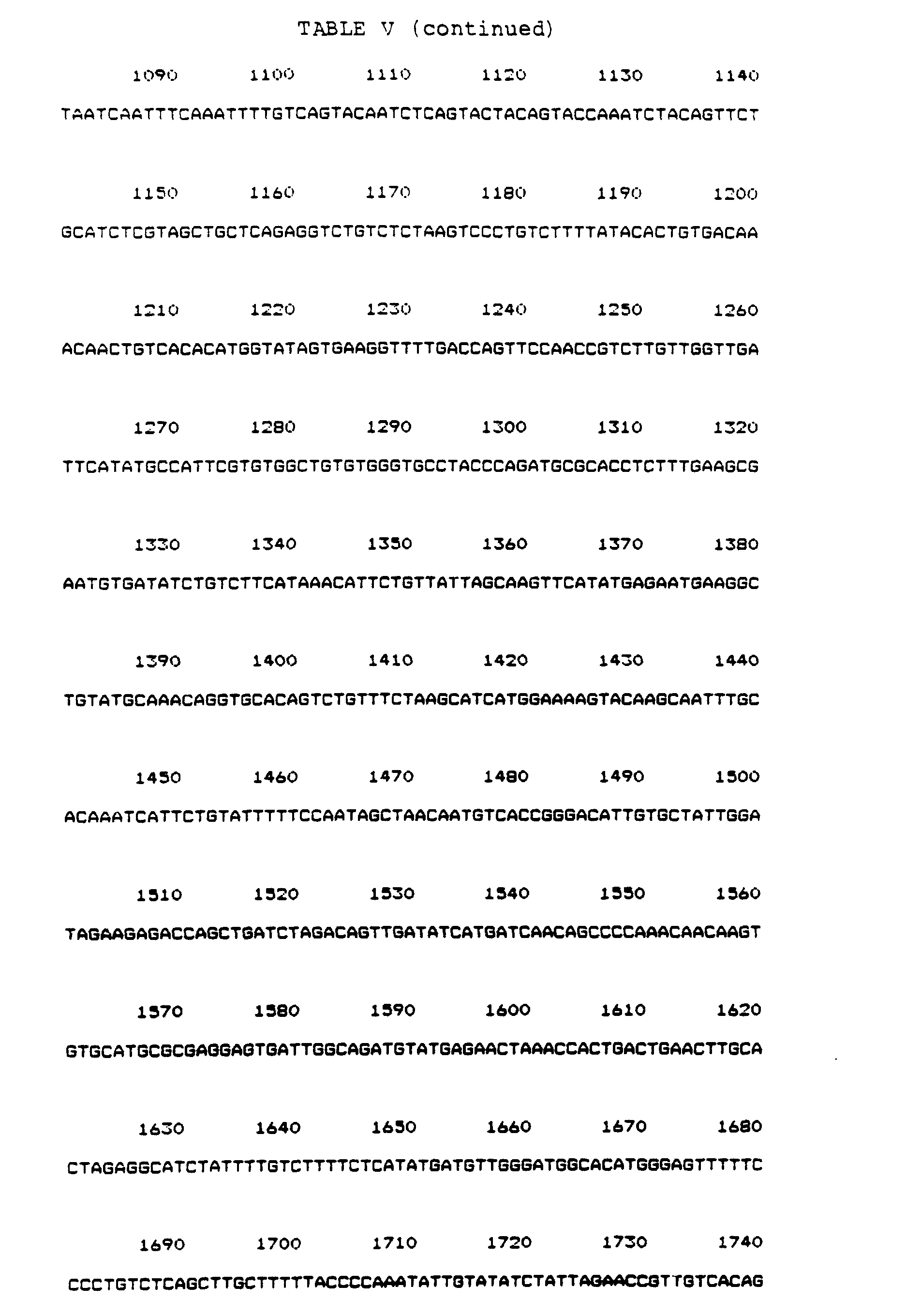

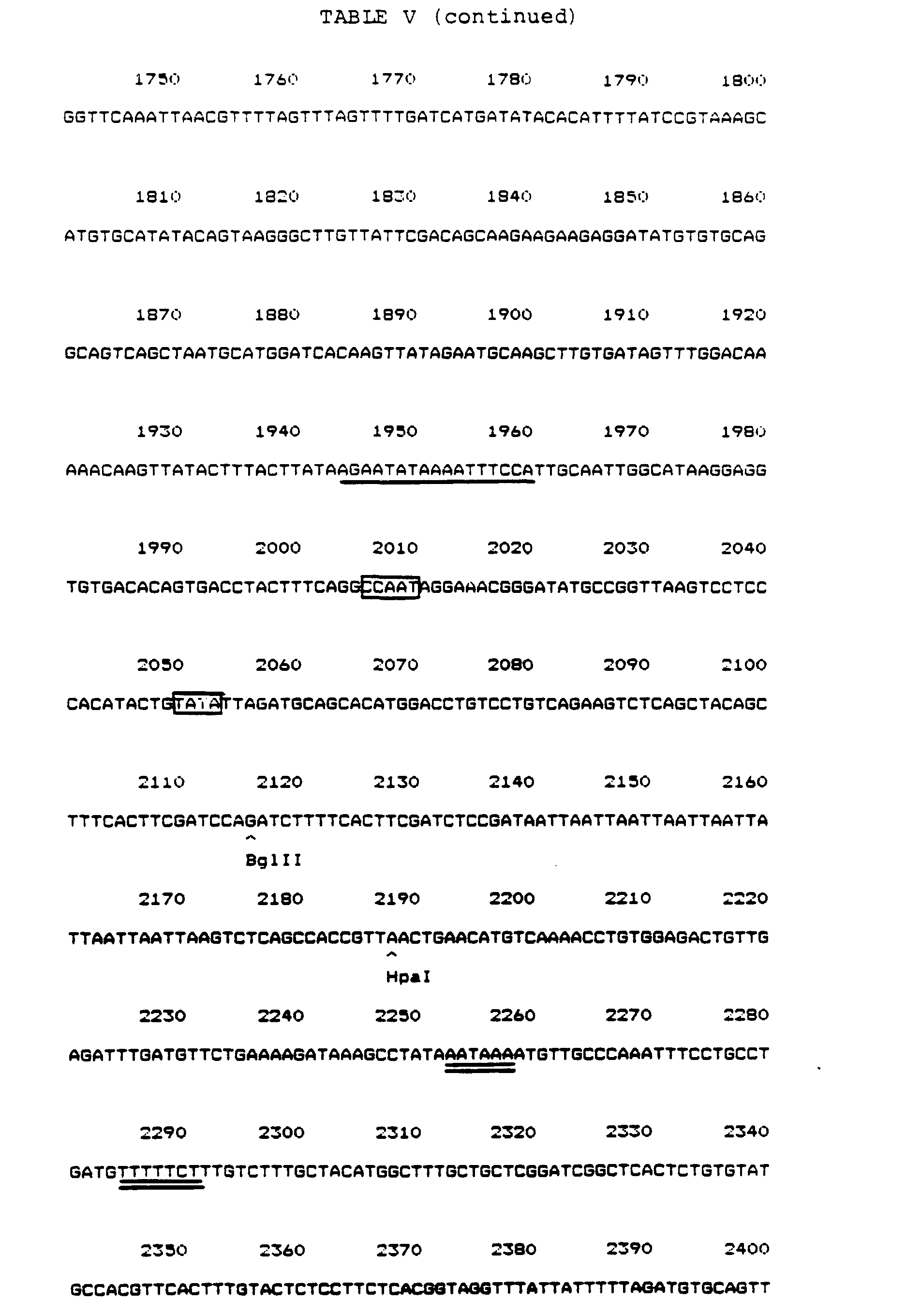

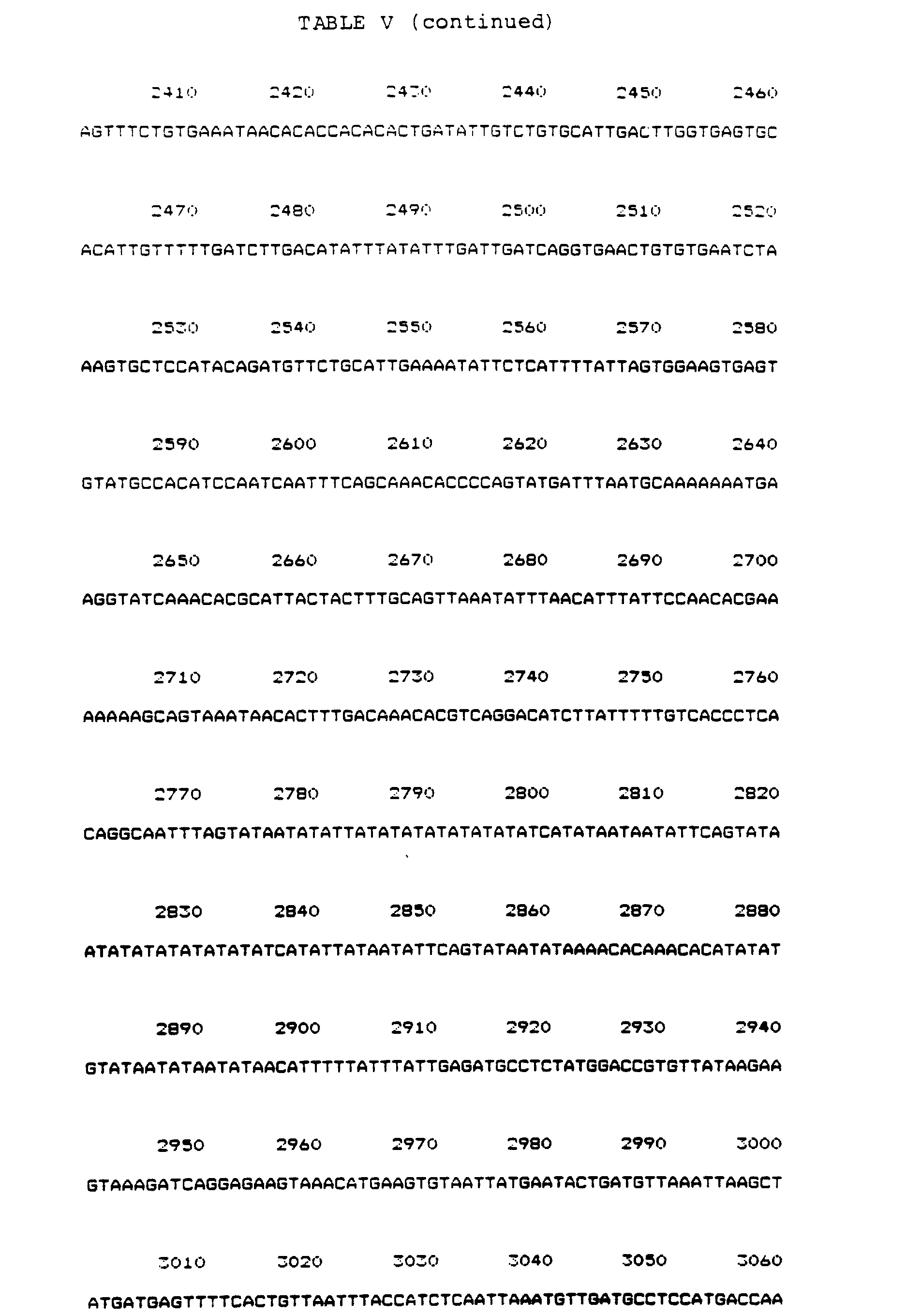

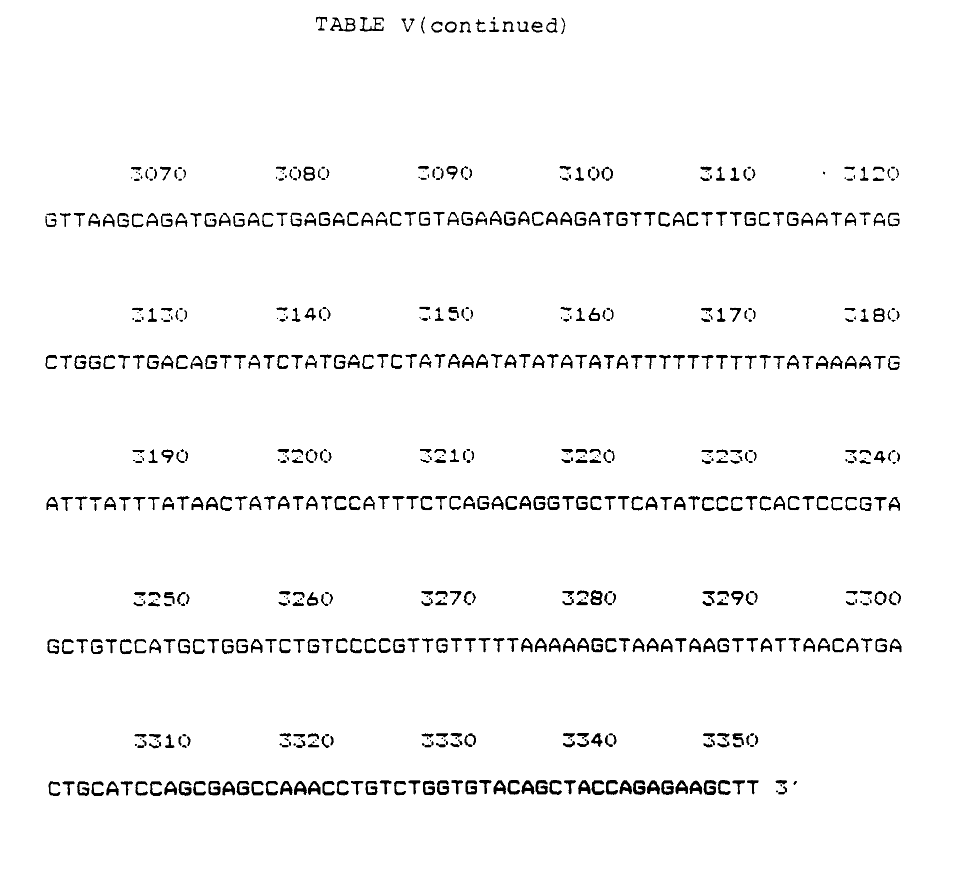

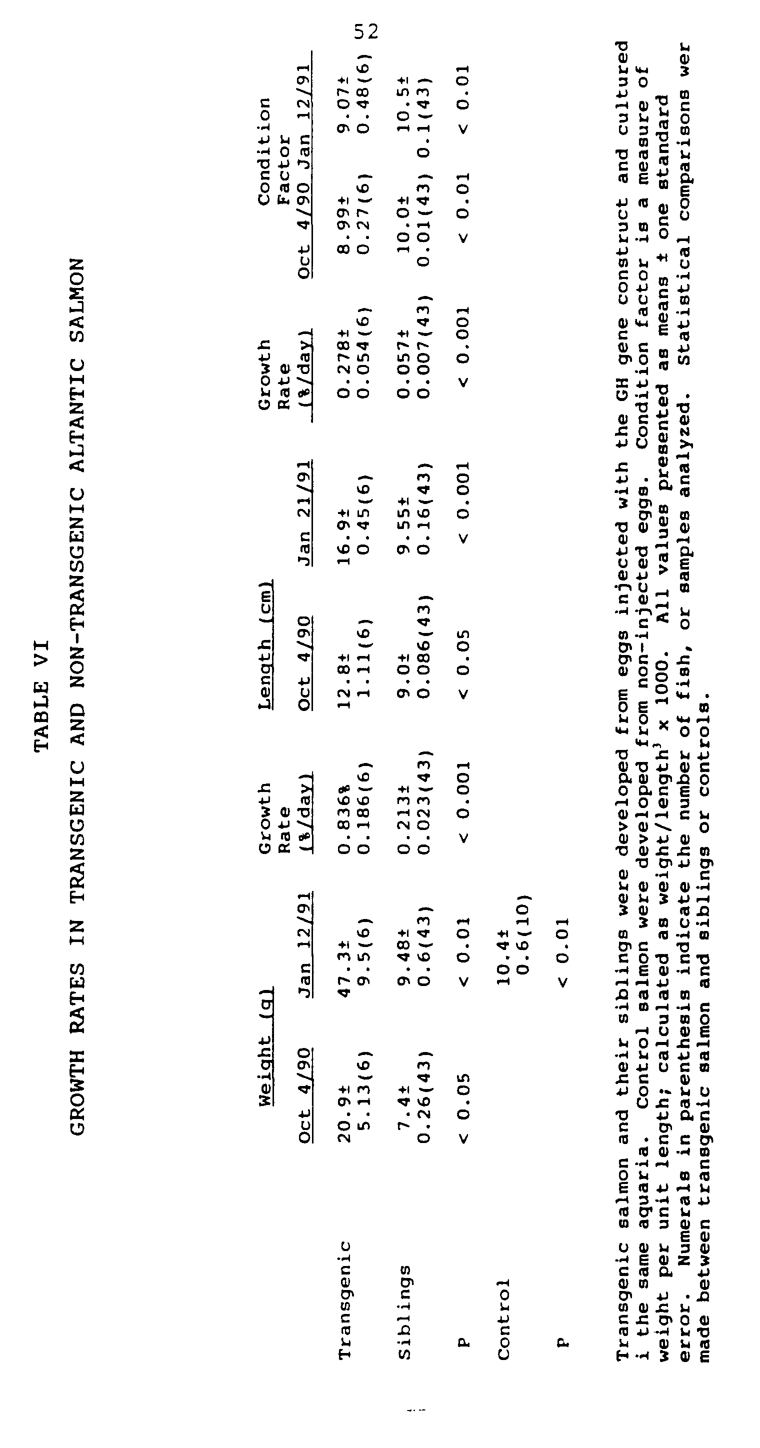

## Claims

1. A transgenic fish characterised by having incorporated in its genome a chimeric gene construct wherein said chimeric gene construct comprises:
a type III AFP promoter; and
a fish growth hormone gene sequence.

2. A transgenic fish as claimed in claim 1, wherein said type III AFP promoter is obtained from a fish selected from the group consisting of ocean pout, wolffish, sea raven and winter flounder.

3. A transgenic fish as claimed in claim 1 or 2, wherein said type III AFP promoter comprises the sequence of Table V from 5' end at base position 1 to base position 2115.

4. A transgenic fish as claimed in any preceding claim, wherein said gene construct comprises the transcriptional terminal sequence of Table V from base pair position 2188 to the 3' end at base pair position 3350.

5. A transgenic fish as claimed in any preceding claim, wherein said chimeric gene construct comprises a combined promoter\transcriptional terminal sequence, which includes a 5' untranslated sequence between said promoter and transcriptional terminal sequence and wherein said combined sequence has the sequence of Table V with unique restriction sites of Bg1II at base pair position 2116 and of HpaI at base pair position 2188.

6. A transgenic fish as claimed in claim 5, wherein said 5' untranslated sequence between sites Bg1II and HpaI is removed.

7. A transgenic fish as claimed in claim 6, wherein a chimeric gene sequence to be expressed is inserted at said Bg1II site or the HpaI site.

8. A transgenic fish as claimed in any preceding claim, wherein said gene construct is expressed predominantly in fish liver.

9. A transgenic fish as claimed in any preceding claim, wherein said fish is salmon, ocean pout, sea raven or wolffish.

10. An assay for determining a transgenic fish as claimed in any preceding claim, comprising the steps of:
I. amplifying by DNA sequence amplification techniques a portion of the fish genome having a DNA sequence which is a portion of the promoter sequence of table V; and
II. detecting presence of the amplified portion to indicate a transgenic fish.

11. An assay as claimed in claim 10, wherein primers used in PCR amplification technique are selected from sequence fragements of Table V, from the 5' end to the BglII site.

12. An assay as claimed in claim 11,wherein said primers are selected from the group consisting of:
Primer A + 27 5' -GTCAGAAGTCTCAGCTACAGC- 3' + 47 sense strand;
Primer B + 861 5' -ATCTCAACAGTCTCCACAGGT- 3' + 881 antisense strand;
Primer C + 161 5' -TCTGCTGATGCCAGTCTTACT- 3' + 181 sense strand; and
Primer D + 339 5' -ACAGAAGTCCAGCAGGAATAT- 3' + 359 antisense strand.

13. A promoter sequence/transcriptional terminal sequence including a 5' untranslated sequence between said promoter and terminal sequences, said combined sequences having the sequence of Table V with unique restriction sites of Bg1II at base pair position 2116 and of HpaI at base pair position 2188.

14. A promoter/terminal sequence as claimed in claim 13, wherein said 5' untranslated sequence between sites Bg1II and HpaI is removed.

15. A promoter/terminal sequence as claimed in claim 13 or 14, wherein a chimeric gene sequence to be expressed is inserted at said Bg1II site or at said HpaI site.

16. A chimeric gene construct comprising a promoter which has the DNA sequence of Table V from 5' at base pair position 1 to base position 2115.

17. A chimeric gene construct comprising a transcriptional terminal sequence wherein said sequence has the DNA sequence of Table V from base pair position 2188 to the 3' at base pair position 3350.

18. A fish host transformed with a chimeric gene as claimed in claims 16 or 17.

19. A host as claimed in claim 18, wherein said transformed host is a fish embryo cell.

## Revendications

1. Un poisson transgénique caractérisé en ce que qu'il incorpore dans son génome une construction de gène chimérique dans lequel ladite construction de gène chimérique comprend :
- un promoteur AFP de type III ; et
- une séquence de gène de l'hormone de croissance de poisson.

2. Un poisson transgénique selon la revendication 1, dans lequel le dit promoteur AFP de type III est obtenu à partir d'un poisson choisi parmi le groupe consistant en tacaud d'océan, loup de mer, hémiptère (*Hemipterus americanus*) et carrelet.

3. Un poisson transgénique selon la revendication 1 ou 2, dans lequel le dit promoteur AFP de type III comprend la séquence du Tableau V à partir de l'extrémité 5' à la base située à la position 1 jusqu'à la base située à la position 2115.

4. Un poisson transgénique selon l'une quelconque des revendications précédentes, dans lequel ladite construction de gène comprend la séquence terminale transcriptionnelle du Tableau V à partir de la paire de bases située à la position 2188 jusqu'à l'extrémité 3' à la paire de bases située à la position 3350.

5. Un poisson transgénique selon l'une quelconque des revendications précédentes, dans lequel ladite construction de gène chimérique comprend un promoteur/une séquence terminale transcriptionnelle associés, qui inclut une séquence non-traduite 5' entre ledit promoteur et la séquence terminale transcriptionnelle et dans laquelle ladite séquence associée possède la séquence du Tableau V avec des sites de restriction uniques pour BglII à la paire de bases située à la position 2116 et pour HpaI à la paire de bases située à la position 2188.

6. Un poisson transgénique selon la revendication 5, dans lequel ladite séquence non-traduite 5' entre les sites BglII et HpaI est éliminée.

7. Un poisson transgénique selon la revendication 6, dans lequel une séquence de gène chimérique destinée à être exprimée est insérée audit site Bg1II ou Hpal.

8. Un poisson transgénique selon l'une quelconque des revendications précédentes, dans lequel ladite construction de gène est exprimée de façon prédominate dans le foie de poisson.

9. Un poisson transgénique selon l'une quelconque des revendications précédentes, dans lequel ledit poisson est un saumon, tacaud d'océan, hémiptère (*Hemipterus americanus*) ou loup de mer.

10. Un test pour déterminer un poisson transgénique selon l'une quelconque des revendications précédentes comprenant les étapes de :
(i) amplification par des techniques d'amplification d'ADN d'une partie du génome de poisson ayant une séquence d'ADN qui est une partie de la séquence du promoteur du Tableau V; et
(ii) détection de la présence de la partie amplifiée pour reconnaître un poisson transgénique.

11. Un test selon la revendication 10, dans lequel les amorces utilisées dans la technique d'amplification PCR sont choisies parmi les fragments de la séquence du Tableau V, à partir de l'extrémité 5' jusqu'au au site Bg1II.

12. Un test selon la revendication 11, dans lequel lesdites amorces sont choisies parmi le groupe consistant en :
- Amorce A + 27 5'-GTCAGAAGTCTCAGCTACAGC-3'-47 du brin signifiant ;
- Amorce B + 861 5'-ATCTCAACAGTCTCCACAGGT-3'-881 du brin anti-sens ;
- Amorce C + 161 5'-TCTGCTGATGCCAGTCTTACT-3'-181 du brin signifiant ; et
- Amorce D + 339 5'-ACAGAAGTCCAGCAGGAATAT-3'-359 du brin anti-sens.

13. Une séquence de promoteur/séquence terminale transcriptionnelle comprenant une séquence non-traduite 5' entre ledit promoteur et les séquences terminales, lesdites séquences associées ayant la séquence du Tableau V avec des sites de restriction uniques pour Bg1II à la paire de bases située à la position 2I16 et pour HpaI à la paire de bases située à la position 2I88.

14. Un promoteur/séquence terminale selon la revendication 13, dans lequel ladite séquence non-traduite 5' entre les sites Bg1II et HpaI est éliminée.

15. Un promoteur/séquence terminale selon la revendication 13 ou 14, dans lequel une séquence de gène chimérique destinée à être exprimée est insérée au dit site Bg1II ou au dit site HpaI.

16. Une construction de gène chimérique comprenant un promoteur qui possède la séquence d'ADN du Tableau V à partir de l'extrémité 5' à la base située à la position 1 jusqu'à la base située à la position 2115.

17. Une construction de gène chimérique comprenant une séquence terminale transcriptionnelle dans laquelle ladite séquence possède la séquence d'ADN du Tableau V à partir de la base située à la position 2188 jusqu'à l'extrémité 3' à la base située à la position 3350.

18. Un hôte qui est un poisson transgénique ayant un gène chimérique selon la revendication 16 ou 17.

19. Un hôte selon la revendication 18, dans lequel ledit hôte transgénique est une cellule d'embryon de poisson.

## Patentansprüche

1. Transgenfisch, dadurch gekennzeichnet, daß in seinem Genom ein chimeres Genkonstrukt eingebaut ist, bei dem das chimere Genkonstrukt
einen Typ III AFP-Promotor; und
eine Gensequenz für ein Fischwachstumshormon umfaßt.

2. Transgenfisch nach Anspruch 1, bei dem der Typ m AFP-Promotor aus einem Fisch erhalten wird, der ausgewählt ist aus der Gruppe, bestehend aus Amerikanischer Aalmutter (ocean pout), Seewolf (wolffish), Kormoran (sea raven) und Amerikanischer Scholle (winter flounder).

3. Transgenfisch nach Anspruch 1 oder 2, bei dem der Typ III AFP-Promotor die Sequenz der Tabelle V vom 5'-Ende bei Basenposition 1 bis zur Basenposition 2115 umfaßt.

4. Transgenfisch nach einem der vorangegangenen Ansprüche, bei dem das Genkonstrukt die Transkriptionsterminalsequenz von Tabelle V von Basenpaarposition 2188 bis zum 3'-Ende bei Basenpaarposition 3350 umfaßt.

5. Transgenfisch nach einem der vorangegangenen Ansprüche, bei dem das chimere Genkonstrukt eine kombinierte Promotor-/Transkriptionsterminalsequenz umfaßt, die eine nichttranslatierte 5'-Sequenz zwischen dem Promotor und der Transkriptionsterminalsequenz einschließt und bei dem die kombinierte Sequenz die Sequenz von Tabelle V mit einzigartigen Restriktionsstellen von Bg1II bei Basenpaarposition 2116 und von HpaI bei Basenpaarposition 2188 aufweist.

6. Transgenfisch nach Anspruch 5, bei dem die nichttranslatierte 5'-Sequenz zwischen den Stellen Bg1II und HpaI entfernt ist.

7. Transgenfisch nach Anspruch 6, bei dem eine zu exprimierende chimere Gensequenz bei der Bg1II-Stelle oder der HpaI-Stelle insertiert ist.

8. Transgenfisch nach einem der vorangegangenen Ansprüche, bei dem das Genkonstrukt vorwiegend in Fischleber exprimiert wird.

9. Transgenfisch nach einem der vorangegangenen Ansprüche, bei dem der Fisch Lachs, Amerikanische Aalmutter (ocean pout), Kormoran (sea raven) oder Seewolf (wolffish) ist.

10. Test zur Bestimmung eines Transgenfischs nach einem der vorangegangenen Ansprüche, der die folgenden Schritte umfaßt:
I. Amplifizieren eines Teils des Fischgenoms mit einer DNA-Sequenz, die ein Teil der Promotorsequenz von Tabelle V ist, durch DNA-Sequenz-Amplifikationsverfahren; und
II. Detektieren des Vorhandenseins des amplifizierten Teils, um einen Transgenfisch anzuzeigen.

11. Test nach Anspruch 10, bei dem die im PCR-Amplifikationsverfahren verwendeten Primer aus Sequenzfragmenten von Tabelle V vom 5'-Ende bis zur BglII-Stelle ausgewählt sind.

12. Test nach Anspruch 11, bei dem die Primer ausgewählt sind aus der Gruppe, bestehend aus
Primer A + 27 5'-GTCAGAAGTCTCAGCTACAGC-3' + 47 sense-Strang;
Primer B + 861 5'-ATCTCAACAGTCTCCACAGGT-3' + 881 antisense-Strang;
Primer C + 161 5'-TCTGCTGATGCCAGTCTTACT-3' + 181 sense-Strang; und
Primer D + 339 5'-ACAGAAGTCCAGCAGGAATAT-3' + 359 antisense-Strang.

13. Eine nichttranslatierte 5'-Sequenz zwischen den Promotor- und Terminalsequenzen einschließende Promotorsequenz/Transkriptionsterminalsequenz, wobei die kombinierten Sequenzen die Sequenz von Tabelle V mit einzigartigen Restriktionsstellen von BglII bei Basenpaarposition 2116 und von HpaI bei Basenpaarposition 2188 aufweisen.

14. Promotor-/Terminalsequenz nach Anspruch 13, bei der die nichttranslatierte 5'-Sequenz zwischen den Stellen BglII und HpaI entfernt ist.

15. Promotor-/Terminalsequenz nach Anspruch 13 oder 14, bei der eine zu exprimierende chimere Gensequenz an der BglII-Stelle oder an der HpaI-Stelle insertiert ist.

16. Chimeres Genkonstrukt, das einen Promotor umfaßt, der die DNA-Sequenz von Tabelle V vom 5'-Ende bei Basenpaarposition 1 bis zur Basenposition 2115 aufweist.

17. Chimeres Genkonstrukt, das eine Transkriptionsterminalsequenz umfaßt, bei der die Sequenz die DNA-Sequenz von Tabelle V von Basenpaarposition 2188 bis zum 3'-Ende bei Basenpaarposition 3350 aufweist.

18. Fischwirt, der mit einem chimeren Gen nach Anspruch 16 oder 17 transformiert ist.

19. Wirt nach Anspruch 18, bei dem der transformierte Wirt eine Fischembryozelle ist.
